(19) Europäisches Patentamt
European Patent Office
Office européen des brevets

(11) Publication number : **0 414 422 B1**

(12) # EUROPEAN PATENT SPECIFICATION

(45) Date of publication of patent specification : **06.04.94 Bulletin 94/14**

(21) Application number : **90308785.6**

(22) Date of filing : **09.08.90**

(51) Int. Cl.$^5$ : **C07D 491/10,** C07D 498/10, A61K 31/54, // (C07D491/10, 317:00, 221:00), (C07D498/10, 263:00, 221:00)

(54) **2-Oxo-1-oxa-8-azaspiro [4,5] decane derivatives, processes for their preparation and pharmaceutical compositions thereof.**

The file contains technical information submitted after the application was filed and not included in this specification

(30) Priority : **10.08.89 HU 409389**

(43) Date of publication of application : **27.02.91 Bulletin 91/09**

(45) Publication of the grant of the patent : **06.04.94 Bulletin 94/14**

(84) Designated Contracting States : **AT BE CH DE DK ES FR GB GR IT LI LU NL SE**

(56) References cited :
**DE-A- 1 928 704**
**DE-A- 2 118 339**
**US-A- 4 255 432**
**JOURNAL OF MEDICINAL CHEMISTRY, vol. 15, no. 11, 1972, pages 1123 - 1128; J. MAILLARD et al.: "Cycloalkane Spiroheterocyclic Compounds. 9. 8-[ 1,2,3,4-Tetrahydro-2-naphthyl]-2-oxo-1-oxa-3,8-d iazaspiro[4.5]decanes and ..."**
**CHEMICAL AND PHARMACEUTICAL BULLETIN, vol. 24, no. 6, 1976, pages 1179 - 1188; Y. NAGAI et al.: "Studies on Psychotropic Agents. I. Synthesis of 3,8-Disubstituted-1-oxa-3,8-diazaspiro[4,5]decan -2,4-dione Derivatives"**
**MODERN PHARMACOLOGY, C.R. CRAIG, R.E. STITZEL, pages 997-1001**

(73) Proprietor : **RICHTER GEDEON VEGYESZETI GYAR R.T.**
**Gyömröi ut 19-21**
**H-1475 Budapest (HU)**

(72) Inventor : **T th, Edit**
**Szabolcska M.U. 1**
**H-1114 Budapest (HU)**
Inventor : **Törley, J zsef**
**Katona J. u. 41**
**H-1137 Budapest (HU)**
Inventor : **Hegedüs, Béla, Dr.**
**Bartok B. u. 82**
**H-1113 Budapest (HU)**
Inventor : **Szporny, Lászl , Dr.**
**Szabolcska M. u. 7**
**H-1114 Budapest (HU)**
Inventor : **Kiss, Béla**
**Gergely u. 48**
**H-1103 Budapest (HU)**
Inventor : **Pálosi, Eva, Dr.**
**Vend u. 21**
**H-1025 Budapest (HU)**
Inventor : **Gro , D ra, Dr.**
**Napraforg u. 17**
**H-1021 Budapest (HU)**
Inventor : **Laszlovszky, István, Dr.**
**Bart k B. u. 16**
**H-1111 Budapest (HU)**
Inventor : **Lapis, Erzsébet, Dr.**
**Abaliget u. 86**
**H-1173 Budapest (HU)**
Inventor : **Sarkadi, Adám, Dr.**
**Thököly ut 85**
**H-1146 Budapest (HU)**
Inventor : **Ambrus András, Dr.**
**Lenin krt. 23**
**H-1073 Budapest (HU)**
Inventor : **Laszy, Judit, Dr.**
**Beregsuász u. 40**
**H-1112 Budapest (HU)**

(74) Representative : **Pett, Christopher Phineas et al**
**Frank B. Dehn & Co. European Patent Attorneys Imperial House 15-19 Kingsway London WC2B 6UZ (GB)**

EP 0 414 422 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to 1-oxa-2-oxo-3,8-diazaspiro [4-5] decane derivatives, processes for their preparation and pharmaceutical preparations thereof.

A number of therapeutically useful 2-oxo-1-oxa-3,8-diazaspiro [4-5] decane derivatives have been described in the literature e.g. C.A. 71, 91359d (1969); C.A. 78, 719668t (1973);C.A. 78, 2387q (1973); C.A. 81, 33153c and 105368b (1974); C.A. 95, 161765e (1981); Chem. Pharm. Bull. 24 (1976) 1179-1188; as well as in the DE patent specifications Nos. 1,928,704, 2,013,729, 2,013, 668, 2,118,339 and 2,163,000; in BE patent specifications Nos. 775,984, 774,170, 786, 631 and 825,444; in the GB patent specification No. 1,100,281; in the published NL patent application No. 7,214,689 as well as in the US patent specifications Nos. 3,555,033, 3,594,386, 4,244,961 and 4,255,432.

We have now developed a novel range of compounds based on this ring system but for which the substituents bound in 4-position and optionally in the 3-position of the spirodecane skeleton are substantially different. According to one aspect of the present invention, there are provided compounds of formula (I)

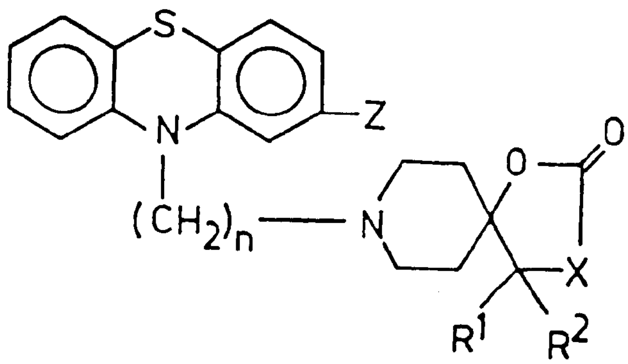

(I)

wherein

X represents an oxygen atom or a NR group, wherein

R represents a hydrogen atom or a $C_{1-12}$ alkyl or $C_{3-6}$cycloalkyl group, or a carbocyclic $C_{6-10}$aryl or carbocylic $C_{6-10}$aryl-$C_{1-4}$alkyl group optionally substituted on the aromatic ring by one or more halogen atoms, $C_{1-4}$ alkyl or $C_{1-4}$alkoxy groups;

$R^1$ and $R^2$ together represent a methylene group or when X is a >NR group, one of $R^1$ and $R^2$ may additionally represent a hydroxyl group and the other represent a methyl group;

Z represents a hydrogen or halogen atom or a trihalomethyl or $C_{2-4}$alkanoyl group, and

n is 2 or 3;

and optical isomers and mixtures thereof and all acid addition and quarternary ammonium salts thereof.

The compounds of formula (I) may exist in various stereoisomeric forms such as geometrical isomers as well as racemates, individual optical isomers and their mixtures, all of which may also occur in the form of various solvates and hydrates. All these compounds and mixtures are within the scope of the present invention.

According to another aspect of the present invention, there is provided a process for the preparation of compounds of the formula (I) and their acid addition and quaternary ammonium salts, which comprises

a) for the preparation of compounds of formula (I) wherein N is a group NR, reacting a 2-oxo-3,8-diazaspiro [4-5] decane derivative of formula (II),

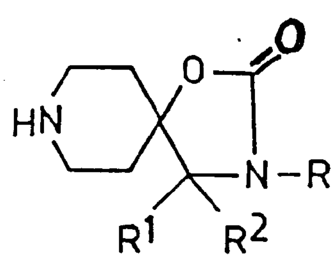

(II)

wherein R,$R^1$ and $R^2$ are as hereinbefore defined with a phenothiazine derivative of the formula (III),

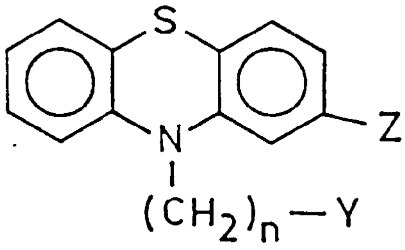

(III)

wherein n and Z are as hereinbefore defined and Y represents a halogen atom or a $C_{1-4}$alkylsulfonyloxy or arylsulfonloxy group;

or

b) for the preparation of compounds of formula (I) as defined in process (a) above, reacting a 2-oxo-3,8-diazaspiro [4,5] decane derivative of formula (IV),

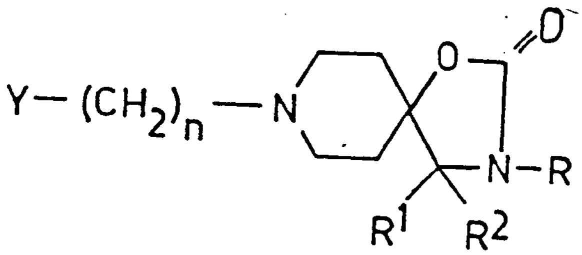

(IV)

wherein R, n, $R^1$, $R^2$ and Y are as defined above with a phenothiazine derivative of formula (V),

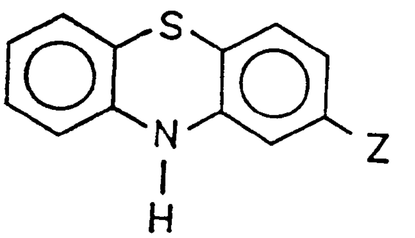

(V)

wherein Z is as defined above;

or

c) for the preparation of compounds of formula (I) wherein $R_1$ and $R_2$ together are a methylene group, reacting a compound of formula (VI)

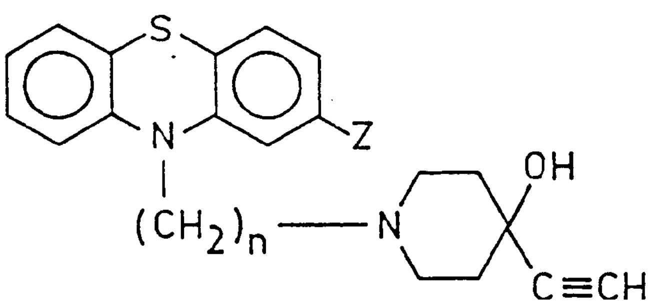

(VI)

wherein n and Z are as defined above, with an isocyanate of formula R-NCO, wherein R is as defined above, to obtain a 4-carbamoyloxy-4-ethynylpiperdine derivative of the formula (VII),

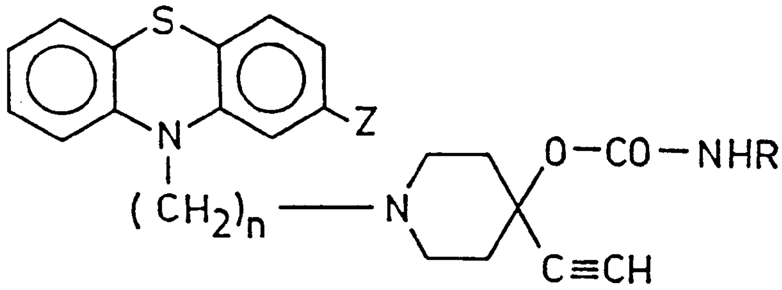

(VII)

wherein R, n and Z are defined above followed by either;

α) for compounds wherein X is a oxygen atom, cyclizing in an acidic medium the compound of formula (VII) as prepared and defined above and reacting the resulting salt formula (VIII),

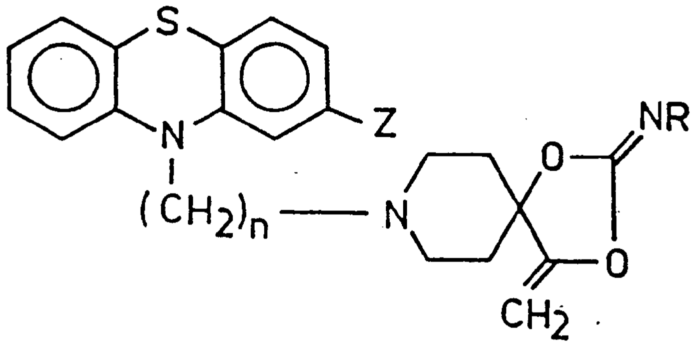

(VIII)

wherein R, n and Z are as defined above with water;

or

β) for compounds wherein X is a NR group, cyclizing in a basic medium the compound of formula (VII) as prepared and defined above; or

d) for the preparation of compunds of formula (I) as defined in process (c) (α) above, cyclizing in an acidic medium a 4-carbamoyloxy-4-ethynylpiperdine derivative of formula (VII) as defined above and reacting the obtained salt of formula (VIII) as defined above with water;

or

e) for the preparation of compounds of formula (I) as defined in process (c) (β) above, cyclizing in a basic medium a 4-carbamoyloxy-4-ethynylpiperidine derivative of formula (VII) as defined above;

or

f) for the preparation of compounds of formula (I) wherein X is a >NR group and one of $R^1$ and $R^2$ is hydroxyl group and the other is a methyl group, reacting a 4-acetyl-4-hydroxypiperidine derivative of formula (X),

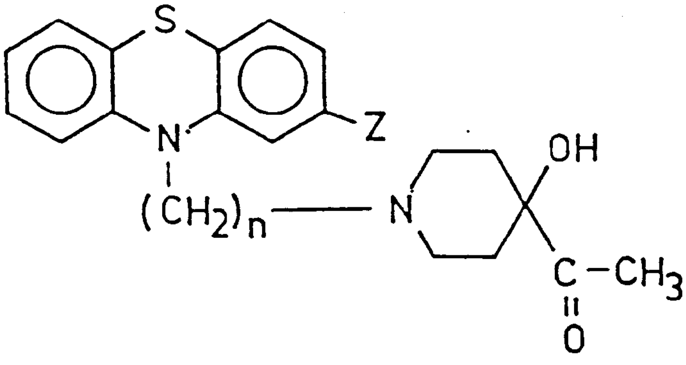

(X)

wherein n and Z are as defined above, with an isocyanate of formula R-NCO, wherein R is as defined above, to obtain a 4-acetyl-4-carbamoyloxypiperidine derivative of the formula (IX),

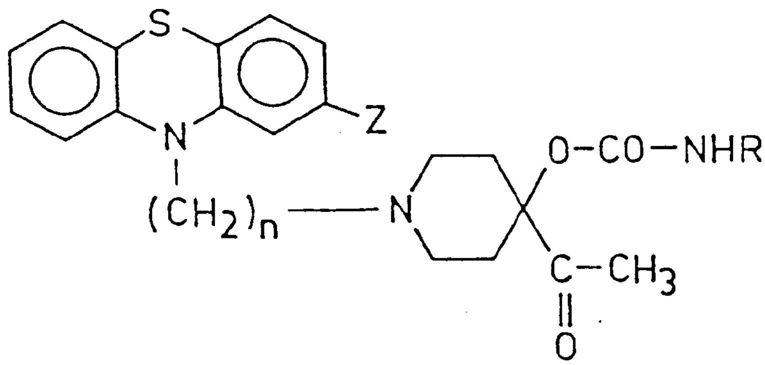

(IX)

wherein R, n and Z are as defined above, which is then cyclized;

or

g) for the preparation of compounds of formula (I) wherein X is a >NR group and one of $R^1$ and $R^2$ is a hydroxyl group and the other is a methyl group, cyclizing a 4-acetyl-4-carbamoyloxypiperdine derivative of formula (IX) as defined above;

followed if desired or necessary,

by reacting a compound of the formula (I) as prepared above, wherein X is an oxygen atom and $R^1$ and $R^2$ together are a methylene group and n and Z are as defined above, with an amine of formula $R\text{-}NH_2$, wherein R is as defined above, in order to prepare a compound of the formula (I), wherein X is a NR group and one of $R^1$ and $R^2$ is a hydroxyl group and the other is a methyl group;

and/or if desired or necessary,

transforming a functional group of a compound of formula (I) as prepared above

into another compound of formula (I) as hereinbefore defined;

and/or if desired or necessary,

reacting a compound of formula (I) as prepared above with an acid to obtain the acid addition salt thereof and/or reacting with a base salt of a compound of formula (I) as prepared above to liberate the free basic form thereof and/or converting a thus prepared compound of formula (I) into its quaternary ammonium salt.

In processes a) and b) above, Y represents a leaving group, e.g. a mesyloxy or tosyloxy group, or a halogen atom, preferably chlorine or bromine. The condensation reaction is preferably carried out in an inert organic solvent and desirably in the presence of a base capable of binding the acid liberated in the reaction. Useful solvents for this purpose include aliphatic alkanols such as ethanol, isopropanol or butanol; aromatic hydrocarbons such as toluene, xylene or benzene; ethers such as dibutyl ether or dioxan; tertiary aliphatic acid amides such as dimethylformamide or dimethylacetamide; ketones such as acetone, methyl ethyl ketone or methyl isobutyl ketone; or any mixture of the above solvents.

Useful acid binding agents include inorganic or tertiary organic bases such as alkaline metal or alkaline earth metal carbonates or hydrogen carbonates, alakaline metal hydroxides, triethyl amine, dimethyl aniline or pyridine; though an excess of the compounds of the formula (II) or (V) may also be used as the acid binding agent. This reaction may be carried at a temperature between room temperature and the boiling point of the reaction mixture, optionally in the presence of a catalyst. Useful catalysts are e.g. alkaline metal iodides. It is suitable to work under an inert gas such as nitrogen or argon during this reaction.

In the first step of process c) above, a 4-ethynyl-4-hydroxypiperidine derivative of the formula (VI) may be brought into reaction with an isocyanate of the formula R-NCO in a manner know per se /Houben-Eyl: Methoden der Organischen Chemie Vol. VIII/3, pages 137 to 147 (1952)/ to give a 4-carbamoyloxy-4-ethynylpiperidine derivative of the formula (VII), which is then cyclized in an acidic or basic medium respectively, according to step α) of β).

For step α), the cyclization may be carried out in an inert anhydrous solvent and in the presence of a suitable acid, preferably in the presence of a dry hydrogen halide. Suitable solvents for this reaction are e.g. aliphatic or alicyclic ethers such as diethyl ether, dipropyl ether, diisopropyl ether, dibutyl ether, tetrahydrofuran or dioxan; or lower aliphatic carboxylic acids such as acetic or propionic acid.

As a hydrogen halide hydrogen chloride, bromide, iodide or fluoride, preferably hydrogen chloride or bromide, may be used. Following the reaction with water, the thus formed 2-imino-1,3-dioxolane hydrohalide salt, the 1-oxa-2-oxo-3,8-diazaspiro [4-5] decane derivative of the formula (I) is obtained as an acid addition salt, from which, if desired, the free base can be liberated in a manner known per se.

The cyclization of 4-carbamoyloxy-4-ethynylpiperidine derivative of the formula (VII) according to step β may be realized in the presence of a base.

Alkaline metal acetates, carbonates, alkoxides, hydroxides and/or tertiary organic bases, e.g. pyridine, tripropylamine or picoline, may be used as basic catalysts in the cyclization; the organic bases may also serve as solvents for the reaction. Further suitable solvents are aliphatic alcohols, e.g. methanol, ethanol, propanol or butanol; aliphatic, alicyclic or aromatic hydrocarbons, e.g. hexane, cyclohexane, benzene, toluene or xylene; acid amides, e.g. dimethylformamide or N-methyl-2-pyrrolidone; ethers such as dibutyl ether or dioxane; nitriles such as acetonitrile; sulfoxides, e.g. dimethylsulfoxide; etc. and any mixture of the above solvents. The reaction may also be carried out without any solvent, e.g. in a molten state. In order to accelerate the cyclization the temperature may be suitably increased. The reaction is preferably accomplished between 40 °C and the boiling point of the reaction mixture. It is preferable to work under an inert gas such as argon or nitrogen. According to a preferred embodiment of the present invention the 4-carbamoyloxy-4-ethynyl-piperidine derivative of the formula (V), formed in the reaction of the 4-ethynyl-4-hydroxypiperidine derivative of the formula (IV) with the isocyanate of the formula R-NCO, is directly cyclized in the same reaction mixture, in the presence of a suitable base, i.e. the derivative of formula (V) is not isolated prior to cyclization.

In the processes d) and e) above the procedures as discussed for steps $\alpha$) and $\beta$) above may be followed.

In the process f) above, a 4-acetyl-4-hydroxypiperidine derivative of the formula (X) is reacted with an isocyanate of the formula R-NCO and the obtained 4-acetyl-4-carbamoyloxypiperidine derivative of the formula (IX) is cyclized. The condensation reaction according to the first step may be realised in a manner known per se [Houben-Weyl: Methoden der Organischen Chemie Vol. VIII/3, pages 137 to 147 (1952)]. The obtained 4-acetyl-4-carbamoyloxypiperidine derivative of the formula (IX) is preferably cyclized in the presence of a base. The cyclization may be carried out under the same reaction conditions as described for step $\beta$) of process c) above. Alternatively, according to a preferred embodiment of process (f) of this invention, the 4-acetyl-4-carbamoyloxypiperidine derivative of the formula (IX) , created in the reaction of the 4-acetyl-4-hydroxypiperidine derivative of the formula (X) with the isocyanate of formula R-NCO, may be cyclized directly in the same reaction mixture, and in the presence of a suitable base ,i.e. the derivative of formula (IX) is not isolated from its reaction mixture before its cyclization.

For process g) above, the procedure in the second step of process f) above may be followed.

If desired or necessary, the compounds of formula (I) as prepared in any one of the processes a) to g) above may be transformed into other compounds within the scope of the formula (I) in a manner known per se.

Thus, on reacting a compound of the formula (I) as prepared above wherein X is an oxygen atom and $R^1$ together with $R^2$ are a methylene group with an amine of formula $R\text{-}NH_2$ wherein R is as defined above, a compound of formula (I) may be prepared wherein X is a >NR group and one of $R^1$ and $R^2$ is a hydroxyl group and the other is a methyl group. This reaction may be carried out in a suitable solvent or without any solvent. Convenient solvents are e.g. aliphatic, alicyclic or araliphatic alcohols such as ethanol, butanol, cyclohexanol, benzyl alcohol; aliphatic or aromatic hydrocarbons such as hexane, heptane, xylene, chlorobenzene or nitrobenzene; ethers, e.g. dioxane; ketones, e.g. di-n-butyl ketone; tertiary organic bases, e.g. picoline, triethylamine or pyridine. An excess of the $R\text{-}NH_2$ amine may also serve as a solvent for this reaction. This procedure may be carried out at a temperature between room temperature and the boiling point of the reaction mixture, preferably under an inert gas, e.g. argon or nitrogen.

If desired or necessary, a compound of the formula (I) wherein $R_1$ and $R_2$ are a hydroxyl and a methyl group may be dehydrated into a compound of formula (I) wherein $R^1$ and $R^2$ together are a methylene group. The dehydration may be achieved under normal or reduced pressure using well-known procedures. Isocyanates, aliphatic carboxylic acids, aliphatic or aromatic carboxylic acid anhydrides, Lewis acids, sulfuric acid or aromatic sulfonic acids may be employed for the dehydration. This reaction is preferably performed in an organic solvent. Suitable solvents are e.g. aromatic hydrocarbons such as benzene, toluene or xylene; ethers such as dioxane, di-n-butyl ether; or aliphatic carboxylic acids such as acetic acid. Optionally, the water formed in the reaction may be removed by azeotropic distillation.

If desired or necessary, the reverse reaction to the dehydration process described above may be carried out by adding water to a compound of formula (I) wherein $R^1$ and $R^2$ together are a methylene group to give a compound of the formula (I) wherein $R_1$ and $R_2$ are a hydroxyl and a methyl group. This hydration reaction may be accomplished in an aqueous medium, in the presence of mineral and/or organic acids. As acids e.g. hydrogen halides, sulfuric, phosphoric, formic acid, aromatic sulfonic acids, oxalic or trifluoroacetic acid may be employed. This reaction may be carried out between 5 °C and the boiling point of the reaction mixture.

The compounds of the formula (I) may be converted into their acid addition salts or quaternary ammonium salts by using methods known per se. For the preparation of acid addition salts, inorganic or organic acids such as hydrogen halides, e.g. hydrochloric acid and hydrobromic acid, sulfuric acid, phosphoric acids as well as formic, acetic, propionic, oxalic, glycolic, maleic, fumaric, succinic, tartaric, ascorbinic, citric, malic, salicylic, lactic, benzoic, cinnamic, aspartic, glutamic, N-acetyl-aspartic or N-acetylglutamic acid as well as alkanesulfonic acids such as methanesulfonic acid or arenesulfonic acids, e.g. p-toluenesulfonic acid may be used.

Salt formation may be carried out e.g. in such a way that the relevant acid is added to the solution of the compound of formula (I) in an inert solvent, e.g. ethanol, and the salt formed is precipitated by adding preferably a water-immiscible organic solvent, e.g. ethyl ether. For the preparation of quaternary ammonium salts a lower alkyl, alkenyl or benzyl halide or an alkyl sulfate may preferably be employed. The quaternization may be performed in an organic solvent such as acetone, acetonitrile, ethanol or their mixtures, at a temperature in the range from room temperature up to the boiling point of the solvent. The acid addition or quaternary ammonium salt obtained may be isolated e.g. by filtration and, when necessary, purified by recrystallization.

Conversely, the corresponding bases can be liberated from their salts by an alkaline treatment.

Among the starting materials, the compounds of the formulae (III) and (V), as well as R-NCO and R-$NH_2$ are known or may be prepared analogously to methods known from the literature.

The compounds of the formulae (III) and (IV) may be prepared e.g. according to J. Heterocycl. Chem. 21, 613 (1984) or J. Med. Chem. 11, 622 (1968) or by using known processes for the alkylation of secondary amines.

The preparation of compounds of formula (II) is described in our EP-A-0412820 of even date, claiming priority from Hungarian patent application No. 4092/89,

This preparation comprises the reduction of compounds of formula (XII)

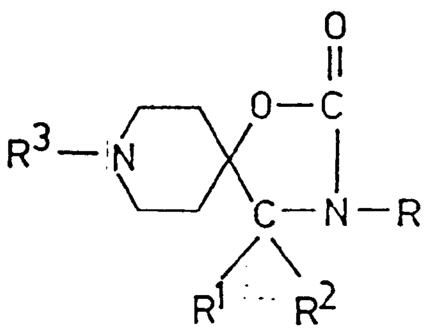

(XII)

wherein $R_1$ and $R_2$ are as defined above and $R^3$ represents a benzyl, ($C_{1-4}$ alkoxy)-carbonyl, phenoxycarbonyl, formyl, piperidin-1-ylcarbonyl, morpholin-4-ylcarbonyl, 4-methylpiperazin-1-ylcarbonyl, 4-(2-hydroxyethyl)piperazin-1-ylcarbonyl, 2-chloro-3-nicotinoylcarbamoyl or $C_{1-6}$alkylcarbamoyl group.

The compounds of formula (XII) may be prepared as also described in the above-mentioned application by:

(i) reacting a compound of formula (XI)

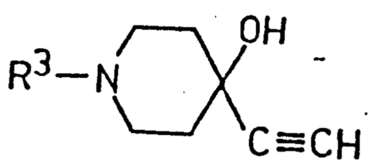

(XI)

wherein $R^3$ is as defined above with an isocyanate of formula RNCO wherein R is as hereinbefore defined followed by cyclizing the resulting compound in a basic medium in order to prepare compounds of formula (XII) wherein $R_1$ and $R_2$ together represent a methylene group; and, if necessary;

(ii) hydrating a compound as prepared in reaction (a) above in order to obtain compounds of formula (XII) wherein one of $R_1$ and $R_2$ is a hydroxy group and the other a methyl group.

Compounds of formula (XI) may be prepared by a procedure analogous to the preparation of compounds of formula (VI) as described below.

The compounds of the formula (VI), (VII), (IX) and (X) as defined above are new and also biologically active.

The compounds of formula (VI) may be prepared e.g. by the ethynylation reaction of suitably substituted 4-piperidone derivatives as described e.g. Hungarian patent specification No. 166,769 or in Farmaco (Pavia) Ed. Sci. 12, 34 (1957).

The carbamates of formulae (VII) and (IX), may be obtained by e.g. reacting a compound of formula (VI) or (X), respectively, with an isocyanate of the formula R-NCO under conditions described above.

The 4-acetyl-4-hydroxypiperidine derivatives of the formula (X) may be prepared by e.g. hydrating the corresponding 4-ethynyl-4-hydroxypiperidine derivatives of the formula (VI) /Houben-Weyl: Methoden der Organischen Chemie Vol. VII/2a, pages 826 to 835 (1973)/ or by the alkaline treatment of the corresponding 4-methylene-2-oxo-1,3-dioxa-8-azaspiro[4,5]decane derivatives of the formula (I) which are prepared as herein described.

The compounds of the formula (I) according to the present invention show psychotropic and antiallergic actions and have a broad spectrum of therapeutical possibilities. Thus, they may be useful e.g. for the treatment of the so-called functional psychoses and organic psychiatric diseases such as dementia, delirium, stimulants-induced psychoses and the like, as well as diskinesias, anxiety, pruritus, nausea, vomitus and intolerable singultation.

Male CFLP mice with a body-weight of 18 to 25 g and male Hannover-Wistar rats with a body-weight of 160 to 180 g were used for the pharmacological investigations. The test compounds were orally administered in a 2% Tween-80 suspension. The pharmacological investigating methods used are described hereinafter.

1. Protective effect against the amphetamine group toxicity on aggregated mice

The method described in Arch. Int. Pharmacodyn. 163, 79 (1966) was used.

The mice were treated with various doses of the test substances. After one hour following the above treatment, the animals were intraperitoneally given 21 mg/kg of d-amphetamine and then the groups consisting of 10 animals each were tightly packed together in plexiglass boses of 15x15x10 cm in size. After 24 hours had elapsed following the amphetamine treatment the surviving animals were counted. The $ED_{50}$ values of the substances were calculated from the percentage of the surviving animals. The $ED_{50}$ value is the dose protecting 50% of the animals from death. The results are summarized in Table 1.

2. Inhibition of the conditioned avoidance response (CAR inhibition) on rats

The method of D. Bovet et al. (Neuropsychopharmacology, ed. R. Rothlin Vol. 2, p. 142, Elsevier Publishing Co. N.Y. 1961) was used.

Male rats weighing 160 to 180 g were conditioned in an automated 6-channel Shuttle box for 10 days. Each daily session consisted of 50 cycles and each one cycle lasted for 40 seconds. Within a single cycle an intermittent light stimulus for 15 seconds as conditioned stimulus, and an electric stimulus of 0.8 mA for 10 seconds as unconditioned stimulus, were used with an intersignal time of 15 seconds. When an animal changed its half-court during the conditioned stimulus period, the stimulus was abolished and the foot-shock was avoided (conditioned avoidance response; abbreviated: CAR). Animals with a performance of at least 80% on the 8th to 10th days of conditioning were considered to be useful for the study. 3 hours before the experiment the animals were treated with various doses of the test substances and the $ED_{50}$ value, i.e. the dose causing an 50% inhibition of the conditioned avoidance response of the animals, was determined. Groups consisting of 10 animals each were used for each dose. The results are summarized in Table 1.

3. Measurement of the spontaneous body temperature

Groups consisting of 10 mice each were orally treated with 30 mg/kg doses of the test substances. After treatment the rectal temperature of the male mice was determined each hour for 5 hours. The average values of temperature decrease related to the body temperature of the untreated control animals (37.1±0.8 °C) 5 hours following treatment are given in Table 1.

4. Inhibition of the aggressive behaviour of the mice

The method described in J. Pharmacol. Expt. Therap. 125, 28 (1959) was used.

Pairs of male mice weighing 20 to 25 each were treated with the test compounds and then placed in cages. 3 hours following the treatment an aggressive behaviour was induced on the animals by using an electric stimulation of 1 mA current intensity. Each group consisted of 5 pairs of mice. The $ED_{50}$ values were calculated from the percentage of animal pairs showing no aggressive behaviour as a result of the treatment. ($ED_{50}$ value is the dose inhibiting the aggressive behaviour on 50% of the animals.)

The results are illustrated in Table 2.

5. Acute toxicity

Rats were treated with various doses of the test compounds. The deaths of the animals were observed for 14 days. The $LD_{50}$ value, i.e. the dose causing the death of half of the animals, was determined from the percentage of the dead animals.

The results are shown in Table 2.

Chlorpromazine /chemically 2-chloro-10-(3-dimethylaminopropyl)phenothiazine/ was used as reference drug in these investigations.

The abbreviations listed hereinafter are used in the Tables:

CPZ = chlorpromazine;

A = 8-[3-(2-chloro-10H-phenothiazin-10-yl)propyl]-3-methyl-4-methylene-2-oxo-1-oxa-3,8-diazaspiro[4,5]decane;

B = 8-[3-(2-chloro-10H-phenothiazin-10-yl)propyl]-3-ethyl-4-methylene-2-oxo-1-oxa-3,8-diazaspiro[4,5]decane;

C = 3-methyl-4-methylene-2-oxo-8-[3-(2-trifluoromethyl-10H-phenothiazin-10-yl)propyl]-1-oxa-3,8-diazaspiro[4,5]decane;

D = 3-ethyl-4-methylene-2-oxo-8-[3-(2-trifluoromethyl-10H-phenothiazin-10-yl)propyl]-1-oxa-3,8-diazaspiro[4,5]decane;

E = [3-(2-chloro-10H-phenothiazin-10-yl)propyl]-4-methylene-2-oxo-3-propyl-1-oxa-3,8-diazaspiro[4,5]decane;

F = 8-[3-(2-chloro-10H-phenothiazin-10-yl)propyl]-3,4-dimethyl-4-hydroxy-2-oxo-1-oxa-3,8-diazaspiro[4,5]decane;

G = 8-[3-(2-chloro-10H-phenothiazin-10-yl)propyl]-3-ethyl-4-hydroxy-4-methyl-2-oxo-1-oxa-3,8-diazaspiro[4,5]decane;

H = 3,4-dimethyl-4-hydroxy-2-oxo-8-[3-(2-trifluoromethyl-10H-phenothiazin-10-yl)propyl]-1-oxa-3,8-diazaspiro[4,5]decane;

I = 4-hydroxy-4-methyl-2-oxo-3-propyl-8-[3-(2-trifluoromethyl-10H-phenothiazin-10-yl)propyl]-1-oxa-3,8-diazaspiro[4,5]decane;

p.o. = orally

amph. = amphetamine (d,1-1-phenyl-2-aminopropane)

tox. = toxicity

CAR = conditioned avoidance response

Table 1

| Compound | Protection against amph. $ED_{50}$ mg/kg p.o. | CAR $ED_{50}$ mg/kg p.o. | Decrease in the spontaneous temperature C° |
|---|---|---|---|
| A | 0.6 | 6.0 | -8.8 |
| B | 1.6 | 1.6 | -7.4 |
| C | 0.7 | 1.9 | -9.9 |
| D | 4.1 | 6.8 | -8.2 |
| E | 1.8 | 10.6 | -5.6 |
| F | 0.7 | 4.6 | -9.1 |
| G | 2.4 | 6.9 | -8.1 |
| H | 0.9 | 3.1 | -9.6 |
| I | 4.2 | 5.6 | -7.0 |
| CPZ | 7.2 | 17.1 | -7.9 |

Table 2

| Compound | Antiaggressive effect, $ED_{50}$ mg/kg p.o. | $LD_{50}$ mg/kg p.o. |
|---|---|---|
| A | 1.7 | 353 |
| B | 10.9 | 500 |
| C | 1.2 | 235 |
| D | 2.5 | 500 |
| E | 2.0 | - |
| CPZ | 4.3 | 225 |

It is clearly obvious from the above Tables that the compounds of the formula (I) according to the present invention possess a significantly stronger effect than that of the reference drug and their therapeutic range is more advantageous than that of CPZ; simultaneously, their effective doses do not induce any sedative, muscle relaxant or anticholinergic action and, even in the case of a chronic treatment, they do not cause the hypersensitization of the dopaminergic system of the central nervous system. Thus, it is likely that their side effects will be diminished in relation to the reference drug in clinical practice. Being effective antipsychotic agents, the compounds of formula (I) according to the present invention are useful for the treatment of psychotic conditions of mammals. The effective doses of course depend on the condition of the patient and on the severity of the disorder to be treated. They may be varied from 0.01 mg/kg to 5 mg/kg of body-weight in the case of oral administration.

If desired, the compounds according to the invention can be formulated into pharmaceutical compositions.

These compositions may be administered in oral, rectal and/or parenteral route. For oral administration, the composition may be formulated e.g. as a tablet, dragée or capsule. In order to prepare oral compositions, e.g. lactose or starch may be used as carriers. Gelatine, sodium carboxymethylcellulose methylcellulose, polyvinylpyrrolidone or starch gum are suitable binding or granulating agents. As disintegrating agents mainly potato starch or microcrystalline cellulose may be added though ultraamylopectin or formaldehyde-casein are also useful.

Talc, colloidal silicic acid, stearin, calcium or magnesium stearate and the like are suitable anti-adhesive and sliding agents.

Tablets may be prepared e.g. by compression following wet granulation. The mixture of the active ingredient with the carriers and optionally with a part of the disintegrating additive is granulated with an aqueous, alcoholic or aqueous-alcoholic solution of the binding agents then the granulate is dried. Subsequently, after mixing the other disintegrating, sliding and anti-adhesive additives to the dried granulate, the mixture is compressed into tablets. If desired, the tablets may be provided with a groove in order to facilitate their administration. Tablets may also directly be prepared from a mixture containing the active ingredient and suitable additives. The tablets may optionally be converted to dragées by employing the commonly used pharmaceutical additives, e.g. protective, flavouring or colouring agents such as sugar, cellulose derivatives (methyl- or ethyl-cellulose, sodium carboxymethylcellulose and the like), polyvinylpyrrolidone, calcium phosphate, calcium carbonate, food dyes, dyeing lacquers, aromatizing agents and iron oxide pigments. Capsulated compositions may be prepared by filling a mixture of the active ingredient with the additives into capsules.

For rectal administration, the composition of the invention may be formulated as a suppository containing a carrier mass, the so-called "adeps pro suppositorio", in addition to the active ingredient. As carriers, vegetable fats such as hardened vegetable oils, or triglycerides of $C_{12-18}$ fatty acids (preferably the carriers bearing the trade name Witepsol) may be used. The active ingredient is uniformly distributed in the molten carrier mass, then suppositories are prepared by moulding.

For parenteral administration, the composition of the invention may be formulated as an injectable solution. For preparing these injectable solutions, the active ingredients are dissolved in distilled water and/or various organic solvents, e.g. glycol ethers, if desired, in the presence of solubilizing agents such as polyoxyethylene sorbitan monolaurate or monooleate or monostearate (Tween 20, Tween 60 or Tween 80), respectively. The injectable solution may further contain various additives (auxiliary agents), e.g. preservatives such as benzylalcohol, methyl p-oxybenzoate, propyl p-oxybenzoate, benzalkonium chloride or phenyl mercury borate; antioxidants such as ascorbinic acid, tokoferole, sodium pyrosulfate; and optionally a complexing agent being capable of binding metal traces, such as ethylenediamine tetraacetate as well as pH-modifying and buffering substances or, if desired, a local anaesthetic agent such as lidocaine. Before filling into the ampoules, the injectable solution containing the composition of the invention is filtered and after filling in, it is subjected to sterilization.

According to a further feature of this invention, the compounds of the invention as defined herein may be used in the manufacture of a medicament for the treatment of psychiatric and allergic diseases, e.g. functional psychoses and organic psychiatric diseases such as dementia, delirium and stimulants-induced psychoses, as well as dyskinesias, anxiety, pruritus, nausea, vomitus and intolerable singultation. This treatment comprises administering a therapeutically effective amount of an active ingredient of the formula (I) or a pharmaceutically acceptable acid addition salt or quaternary ammonium salt thereof to the patient.

The invention is illustrated by way of the following preparations and Examples.

## Preparation 1

4-ethynyl-4-hydroxy-1-[3-(2-trifluoromethyl-10H-phenothiazin-10-yl)propyl]piperidine

Gaseous acetylene is bubbled through a solution of 7.7 g of potassium tertiary butoxide in 46 ml of tetrahydrofuran at 0 to -5 °C for 30 minutes under stirring. Thereafter, the solution containing 18.4 g of 1-[3-(2-trifluoromethyl-10H-phenothiazin-10-yl)propyl]-4-piperidone in 40 ml of tetrahydrofuran is added dropwise and acetylene is introduced for an additional one hour. Then, the reaction mixture is decomposed by adding aqueous saturated ammonium chloride solution under nitrogen at 0 °C and the solvent is evaporated under reduced pressure. The evaporation residue is extracted with benzene, the benzene solution is washed with water to neutral, dried over anhydrous sodium sulfate and evaporated under reduced pressure. The crude title product obtained as evaporation residue is recrystallized from diisopropyl ether under clarifying by activated carbon to give the crystalline title compound in 89% yield, m.p.: 94-96 °C.

Analysis:

calculated for $C_{23}H_{23}F_3N_2OS$:

C 63.87; H 5.36; F 13.18; N 6.48; S 7.41%;

found: C 63.84; H 5.40; F 13.31; N 6.50; S 7.58%.

Using the appropriate starting materials the following compounds may be prepared in an analogous manner to the process described above.

4-Ethynyl-4-hydroxy-[3-(2-chloro-10H-phenothiazin-10-yl)propyl]pipridine, m.p.: 113-114 °C; and

4-Ethynyl-4-hydroxy-1-[3-(10H-phenothiazin-10-yl)propyl]piperidine, m.p.: 118-120 °C.

Preparation 2

4-butylcarbamoyloxy-4-ethynyl-1-[3-(2-trifluoromethyl-10H-phenothiazin-10-yl)propyl]piperidine

A mixture containing 21.6 g of 4-ethynyl-4-hydroxy-1-[3-(2-trifluoromethyl-10H-phenothiazin-10-yl)propyl]piperidine, 7.4 ml of butyl isocyanate and 100 ml of triethylamine is gently refluxed under nitrogen while stirring for 7 to 8 hours, then the reaction mixture is evaporated under reduced pressure. After clarifying the evaporation residue by activated carbon in methanol, the solution is evaporated under reduced pressure. The crude product obtained as evaporation residue is recrystallized from diisopropyl ether under clarifying by activated carbon to give the crystalline title compound in 74% yield, m.p.: 109-110 °C.

Analysis:

calculated for $C_{28}H_{32}F_3N_2O_2S$:

C 63.25; H 6.07; F 10,72; N 7.90; S 6.303;

found: C 63.11; H 6.26; F 10.66; N 7.71; S 6.07%.

Using the appropriate starting materials the following compound may be prepared in an analogous manner to the process described above.

4-Acetyl-4-phenylcarbamoyloxy-1-[3-(2-trifluoromethyl-10H-phenothiazin-10-yl)propyl]piperidine, m.p.: 156-157 °C.

Preparation 3

4-acetyl-4-hydroxy-1-[3-(2-trifluoromethyl-10H-phenothiazin-10-yl)propyl]piperidine

A solution containing 9.5 g of 4-methylene-2-oxo-8-[3-(2-trifluoromethyl-10H-phenothiazin-10-yl)propyl]-1,3-di-oxa-8-azaspiro[4,5]decane (Example 10 below) in 50 ml of benzene is vigorously stirred with 100 ml of 10% aqueous sodium hydroxide solution at 70 to 80 °C under argon. After termination of the reaction (which is observed by using thin layer chromatography) the heterogeneous reaction mixture is cooled down. After separation the benzene layer is washed with water to neutral, dried over anhydrous magnesium sulfate and evaporated under reduced pressure. The evaporation resiude is recrystallized from isopropyl ether to give the title compound in 56% yield, m.p.: 75-76 °C.

Analysis:

calculated for $C_{23}H_{25}F_3N_2O_2S$:

C 61.31; H 5.59; F 12.65; N 6.22; S 7.12;

found: C 61.33; H 5.70; F 12.60; N 6.11; S 7.00.

Preparation 4

4-methylene-2-oxa-3-n-propyl-1-oxa-3,8 diazaspiro [4,5] decane

(a) 8-benzyl-3-n-butyl-4-methylene-2-oxo-1-oxa-3,8- diazaspiro[4,5]decane

21.5 g of 1-benzyl-4-ethynyl-4-hydroxypiperidine are boiled under reflux with 12.9 g of n-butyl isocyanate in the presence of 0.4 g of anhydrous potassium acetate in 66 ml of 2-picoline under nitrogen for 6 hours. After evaporating 2-picoline under reduced pressure and dissolving the residue in benzene, the organic solution is washed with water and dried over anhydrous magnesium sulfate. After filtration on an aluminum oxide layer the benzene solution is evaporated under reduced pressure. The crude product obtained is recrystallized from n-heptane to give the pure title substance in 78.5% yield, m.p.: 57-58 °C.

Analysis:

Calculated for $C_{19}H_{26}N_2O_2$

| | | | |
|---|---|---|---|
| | C 72.58; | H 8.33; | N 8.91%; |
| found: | C 72.55; | H 8.53; | N 9.06%. |

Using the appropriate starting materials the following compounds may be prepared in an analogous manner;

8-Formyl-4-methylene-2-oxo-3-phenyl-1-oxa-3,8-diazaspiro[4,5]decane, m.p.: 171-172 °C;

8-Benzyloxycarbonyl-4-methylene-2-oxo-3-phenyl-1-oxa-3,8-diazaspiro[4,5]decane, m.p.: 145-146 °C;

4-Methylene-2-oxo-3-phenoxycarbonyl-3-phenyl-1-oxa-3,8-diazaspiro[4,5]decane, m.p.: 208-210 °C;

8-Benzyl-3-cyclohexyl-4-methylene-2-oxo-1-oxa-3,8-diazaspiro[4,5]decane, m.p.: 128-130 °C (the hydrogen fumarate salt melts at 207-208 °C);

8-Benzyl-3-ethyl-4-methylene-2-oxo-1-oxa-3,8-diazaspiro[4,5]decane, m.p.: 103-104 °C (hydrogen maleate salt m.p.: 184-186 °C);

8-Benzyl-3-tert-butyl-4-methylene-2-oxo-1-oxa-3,8-diazaspiro[4,5]decane, m.p.: 116-117 (dihydrogen citrate salt m.p.: 132-133 °C);

8-Benzyl-4-methylene-2-oxo-3-phenyl-1-oxa-3,8-diazaspiro[4,5]decane, m.p.: 134-135 °C;

8-Benzyl-3-isopropyl-4-methylene-2-oxo-1-oxa-3,8-diazaspiro[4,5]decane, m.p. 96-97 °C;

8-Benzyl-3-methyl-4-methylene-2-oxo-1-oxa-3,8-diazaspiro[4,5]decane hydrogen maleate, m.p.: 210-211 °C;

8-Benzyl-4-methylene-2-oxo-3-propyl-1-oxa-3,8-diazaspiro[4,5]decane dihydrogen citrate, m.p.: 168-171 °C;

(b)3-tert-butyl-4-methylene-2-oxo-8-phenoxycarbonyl-1-oxa-3,8-diazaspiro[4,5]decane

A solution of 3.6 g of phenyl chloroformate in 5 ml of methylene chloride are dropped into a solution of 6.3 g of 8-benzyl-3-tert-butyl-4-methylene-2-oxo-1-oxa-3,8-diazaspiro[4,5]decane in 30 ml of methylene chloride under argon at 0 °C while stirring, then the reaction mixture is stirred at room temperature for one additional hour. After termination of the reaction the mixture is diluted with 35 ml of methylene chloride, extracted with 4 N sodium hydroxide solution and washed to neutral with water. After drying over anhydrous magnesium sulfate the solvent is evaporated under reduced pressure. After adding n-hexane to the residue the solid precipitate is filtered off and recrystallized from isopropanol to obtain the title substance in 82% yield, m.p.: 125-126 °C.

Analysis:

Calculated for $C_{19}H_{24}N_2O_4$

C 66.26; H 7.02; N 8.13%;

found: C 66.33; H 7.10; N 8.10%.

Using the appropriate starting materials the following compounds may be prepared in an analogous manner.

3-Benzyl-8-benzyloxycarbonyl-4-methylene-2-oxo-1-oxa-3,8-diazaspiro[4,5]decane, oil;
8-Benzyloxycarbonyl-3-butyl-4-methylene-2-oxo-1-oxa-3,8-diazaspiro[4,5]decane, m.p.: 47-48 °C;
8-ethoxycarbonyl-3-methyl-4-methylene-2-oxo-1-oxa-3,8-diazaspiro[4,5]decane, m.p.: 121-122 °C;
3-(3,4-dichlorophenyl)-4-methylene-2-oxo-8-phenoxycarbonyl-1-oxa-3,8-diazaspiro[4,5]decane, m.p.: 220-222 °C;
3-methyl-4-methylene-2-oxo-8-phenoxycarbonyl-1-oxa-3,8-diazaspiro[4,5]decane, m.p.: 118-119 °C; and
4-methylene-2-oxo-8-phenoxycarbonyl-3-propyl-1-oxa-3,8-diazaspiro[4,5]decane, m.p. : 96-98 °C;

c) 4-methylene-2-oxo-3-n-propyl-1-oxa-3,8-diazaspiro[4,5]decane

A suspension containing 0.5 g of 10% by weight palladium-on-carcoal catalyst in 5 ml of water is added to the solution of 5.0 g of 8-benzyloxycarbonyl-4-methylene-2-oxo-3-n-propyl-1-oxa-3,8-diazaspiro[4,5]decane in 45 ml of methanol at 0 °C under argon while stirring. To this mixture 1 ml of 45.8% aqueous hydrazine solution is introduced and the reaction mixture is refluxed for 10 to 15 minutes. After cooling down to room temperature and filtering off the catalyst the solvent is evaporated under reduced pressure and the crude evaporation residue is recrystallized from benzene to give the title compound in 95% yield, m.p.: 96-97 °C.

Using the appropriate starting materials the following compounds may be prepared in an analogous manner;

3-Ethyl-4-methylene-2-oxo-1-oxa-3,8-diazaspiro[4,5]decane, m.p.: 106-108 °C;
3-isopropyl-4-methylene-2-oxo-1-oxa-3,8-diazaspiro[4,5]decane, m.p.: 151-152 °C;
4-methylene-3-(1-naphthyl)-2-oxo-1-oxa-3,8-diazaspiro[4,5]decane, m.p.: 208-209 °C;
3-butyl-4-methylene-2-oxo-1-oxa-3,8-diazaspiro[4,5]decane, oil;
4-methylene-2-oxo-3-phenyl-1-oxa-3,8-diazaspiro[4,5]decane, m.p: 185-186 °C;
3-tert-Butyl-4-methylene-2-oxo-1-oxa-3,8-diazaspiro[4,5]decane, m.p.: 138-139 °C;
3-heptyl-4-hydroxy-4-methyl-2-oxo-1-oxa-3,8-diazaspiro[4,5]decane, m.p.: 139-140 °C;
3-[2-(3,4-dimethoxyphenyl)ethyl]-4-hydroxy-4-methyl-2-oxo-1-oxa-3,8-diazaspiro[4,5]decane, m.p,: 190-191 °C;
3-benzyl-4-methylene-2-oxo-1-oxa-3,8-diazaspiro[4,5]decane, m.p.: 77-79 °C;
3-heptyl-4-methylene-2-oxo-1-oxa-3,8-diazaspiro[4,5]decane, oil;
3-decyl-4-methylene-2-oxo-1-oxa-3,8-diazaspiro[4,5]decane, oil;
3-cyclohexyl-4-methylene-2-oxo-1-oxa-3,8-diazaspiro[4,5]decane, m.p.: 141-142 °C;
3-[2-(3,4-dimethoxyphenyl)ethyl]-4-methylene-2-oxo-1-oxa-3,8-diazaspiro[4,5]decane, m.p.: 107-109 °C.

Preparation 5

3-methyl-4-methylene-2-oxo-1-oxa-3,8-diazaspiro[4,5]decane

2 g of catalyst containing 10% by weight of palladium on charcoal are suspended in 20 ml of water and added to the solution of 20.0 g of 8-benzyl-3-methyl-4-methylene-2-oxo-1-oxa-3,8-diazaspiro[4,5]decane (Preparation 4(a)) in 180 ml of methanol at 0 to 5°C under nitrogen while stirring. Thereafter, 4.9 ml of an aqueous hydrazine solution of 48 g/100 ml concentration are introduced to the mixture which is then gently boiled under reflux. The progress of the reaction is followed by thin layer chromatography (TLC). After termination of the reaction (10 to 15 minutes) the mixture is cooled down, the catalyst is filtered off and washed with methanol. The methanolic washings are combined with the methanolic solution and the solvent is evaporated under reduced pressure. After recrystallizing the evaporation residue from a mixture of ethyl acetate with isopropyl ether the title compound is obtained in 94% yield, m.p.: 92-93 °C.

Analysis:

Calculated for $C_9H_{14}N_2O_2$

C 59.32; H 7.74; N 15.37%;

found: C 59.55; H 7.76; N 15.49%.

Example 1

3-methyl-4-methylene-2-oxo-8-[3-(2-trifluoromethyl-10H-phenothiazin-10-yl)propyl]-1-oxa-3,8-diazaspiro[4,5]decane

A mixture containing 11.0 g of 3-methyl-4-methylene-2-oxo-1-oxa-3,8-diazaspiro[4,5]decane, 41.3 g of 3-(2-trifluoromethyl-10H-phenothiazin-10-yl)propyl chloride, 16.6 g of anhydrous potassium carbonate and 0.6 g of potassium iodide in 110 ml of methyl isobutyl ketone is refluxed under nitrogen while stirring for 6 hours. After evaporating the solvent under reduced pressure benzene and water are added to the evaporation residue, the organic phase is separated, washed with water to neutral, and dried over anhydrous sodium sulfate Then the solution is evaporated under reduced pressure. The solid residue is boiled with hexane, and, after cooling down, the precipitate is filtered off and recrystallized from ethanol to give the title compound in a yield of 75.3%; m.p.: 116-117.5 °C.

Analysis:

Calculated for $C_{25}H_{26}F_3O_2S$:

C 61.33; H 5.35; F 11.64; N 8.58; S 6.55%;

found: C 61.50; H 5.38; F 11.38; N 8.34; S 6.34%.

Example 2

8-[3-(2-chloro-10H-phenothiazin-10-yl)propyl]-4-hydroxy-4-methyl-2-oxo-1-oxa-3,8-diazaspiro[4,5]decane

9.7 g of 8-[3-(2-chloro-10H-phenothiazin-10-yl)propyl]-4-methylene-2-oxo-3-propyl-1-oxa-3,8-diazaspiro[4,5]decane are stirred with 120 ml of a 0.5 mol/litre hydrochloric acid solution at 5 to 10 °C for 3 hours, then the reaction mixture is made alkaline by adding sodium hydroxide solution and extracted with chloroform. The chloroform phase is washed with water to neutral, dried over anhydrous magnesium sulfate and evaporated under reduced pressure. After recrystallization of the evaporation residue from benzene the title compound is obtained in 88% yield, m.p.: 174-176 °C.

Analysis:

calculated for $C_{26}H_{32}ClN_3O_3S$:

C 62.19; H 6.42; Cl 7.06; N 8.37; S 6.39%;

found: C 62.10; H 6.58; Cl 6.91; N 8.40; S 6.60%;

On treating an ethanolic solution of the base with ethereal hydrogen chloride solution the hydrochloride of the above compound may be obtained m.p.: 157-160 °C (with decomposition).

Example 3

8-[3-(2-chloro-10H-phenothiazin-10-yl)propyl]-3-cyclohexyl-4-hydroxy-4-methyl-2-oxo-1-oxa-3,8-diazaspiro[4,5]decane hydrochloride

5.3 g of 8-[3-(2-chloro-10H-phenothiazin-10-yl)propyl]-3-cyclohexyl-4-methylene-2-oxo-1-oxa-3,8-

diazaspiro[4,5]decane are dissolved in 5.3 ml of 98% formic acid at room temperature and 53 ml of 3 mol/litre hydrochloric acid solution are added dropwise under stirring over 20 minutes. The precipitate is filtered off, washed with water and dried to give the title hydrochloride in 98% yield, m.p.: 246-248 °C (with decomposition). The base can be liberated from the hydrochloride by adding aqueous ammonium hydroxide solution.

Analysis of the base:

calculated for $C_{29}H_{36}ClN_3O_3S$:

C 64.25; H 6.69; Cl 6.54; N 7.75; S 5.91%;

found: C 64.28; H 6.82; Cl 6.63; N 7.57; S 6.12%;

Using the appropriate starting materials the following compounds may be prepared in an analogous manner to the processes described in Examples 2 or 3 above or Example 12, described hereinafter.

8-[3-(2-Acetyl-10H-phenothiazin-10-yl)propyl]-3-decyl-4-hydroxy-4-methyl-2-oxo-1-oxa-3,8-diazaspiro[4,5]decane hydrochloride, m.p. 154-157 °C (with decomposition);

8-[3-(2-Chloro-10H-phenothiazin-10-yl)propyl]-3,4-dimethyl-4-hydroxy-2-oxo-1-oxa-3,8-diazaspiro[4,5]decane, m.p.: 172-173 °C; the hydrochloride decomposes at 232-235 °C;

8-[3-(2-Chloro-10H-phenothiazin-10-yl)propyl]-4-hydroxy-3-isopropyl-4-methyl-2-oxo-1-oxa-3,8-diazaspiro[4,5]decane, m.p.: 108-110 °C; the hydrochloride decomposes at 127-130 °C;

4-Hydroxy-4-methyl-2-oxo-3-phenyl-8-[3-(2-trifluoromethyl-10H-phenothiazin-10-yl)propyl]-1-oxa-3,8-diazaspiro[4,5]decane hydrochloride, m.p.: 147-150 °C (with decomposition);

3-Ethyl-4-hydroxy-4-methyl-2-oxo-8-[3-(2-trifluoromethyl-10H-phenothiazin-10-yl)propyl]-1-oxa-3,8-diazaspiro[4,5]decane, m.p.: 148-149 °C; the hydrochloride decomposes at 186-189 °C; and

4-Hydroxy-4-methyl-2-oxo-8-[3-(10H-phenothiazin-10-yl)propyl]-3-tert-butyl-1-oxa-3,8-diazaspiro[4,5]decane, m.p.: 169-171 °C.

Example 4

8-[3-(2-acetyl-10H-phenothiazin-10-yl)propyl]-3-methyl-4-methylene-2-oxo-1-oxa-3,8-diazaspiro[4,5]decane

A mixture containing 7.3 g of 3-methyl-4-methylene-2-oxo-1-oxa-3,8-diazaspiro[4,5]decane, 9 ml of anhydrous triethylamine, 22.0 g of 3-(2-acetyl-10H-phenothiazin-10-yl)propyl bromide and 80 ml of methyl isobutyl ketone is refluxed under argon while stirring for 6 hours. After cooling down the organic phase is washed with water, dried over anhydrous magnesium sulfate and evaporated under reduced pressure. The crude product obtained is purified by chromatography on a silica gel column by using first chloroform and then ethyl acetate as eluating agent. After combining the ethyl acetate eluate is evaporated under reduced pressure and the residue is recrystallized from ethanol to give the title compound in 84.5% yield, m.p.: 105-106 °C.

Analysis:

calculated for $C_{26}H_{29}N_3O_3S$:

C 67.36; H 6.30; N 9.06; S 6.92%;

found: C 67.48; H 6.51; N 9.01; S 7.11%.

Using the appropriate starting materials the following substances may be prepared in an analogous manner to the processes described in Examples 1 or 4 above or Example 14, described hereinafter.

8-[3-(2-Acetyl-10H-phenothiazin-10-yl)propyl]-3,4-dimethyl-4-hydroxy-2-oxo-1-oxa-3,8-diazaspiro[4,5]decane hydrochloride, m.p.: 165-168 °C (with decomposition);

8-[3-(2-Chloro-10H-phenothiazin-10-yl)propyl]-3-methyl-4-methylene-2-oxo-1-oxa-3,8-diazaspiro[4,5]decane, m.p.: 142-143 °C;

4-Methylene-2-oxo-8-[3-(10H-phenothiazin-10-yl)propyl]-3-n-propyl-1-oxa-3,8-diazaspiro[4,5]decane, m.p.: 98-99 °C;

3-Cyclohexyl-4-methylene-2-oxo-8-[3-(10H-phenothiazin-10-yl)propyl]-1-oxa-3,8-diazaspiro[4,5]decane, m.p.: 159-160 °C;

4-Methylene-2-oxo-8-[3-(10H-phenothiazin-10-yl)propyl]-2-oxo-3-tert-butyl-1-oxa-3,8-diazaspiro[4,5]decane, m.p.: 96-97 °C;

8-[3-(2-Chloro-10H-phenothiazin-10-yl)propyl]-3-isopropyl-4-methylene-2-oxo-1-oxa-3,8-diazaspiro[4,5] decane, m.p.: 162-163 °C;

4-Methylene-2-oxo-3-propyl-8-[3-(2-trifluoromethyl-10H-phenothiazin-10-yl)propyl]-1-oxa-3,8-diazaspiro [4,5]decane, m.p.: 119-121 °C;

8-[3-(2-Acetyl-10H-phenothiazin-10-yl )propyl]-3-n-decyl-4-methylene-2-oxo-1-oxa-3,8-diazaspiro[4, 5]decane hydrogen maleate, m.p.: 108-110 °C;

3-Methyl-4-methylene-2-oxo-8-[3-(10H-phenothiazin-10-yl)pro pyl]-1-oxa-3,8-diazaspiro[4,5]decane, m.p.: 132-133 °C, obtained by reacting 3-methyl-4-methylene-2-oxo-1-oxa-3,8-diazaspiro[4,5]decane with 3-(10-phenothiazin-10-yl)propyl p-toluene-sulfonate;

8-[3-(2-Chloro-10H-phenothiazin-10-yl)propyl]-3-n-butyl-4-methylene-2-oxo-1-oxa-3,8-diazaspiro[4, 5]decane, m.p.: 100-101 °C;

8-[3-(2-Chloro-10H-phenothiazin-10-yl)propyl]-3-cyclohexyl-4-methylene-2-oxo-1-oxa-3,8-diazaspiro [4,5]decane, m.p.: 177-178 °C;

8-[3-(2-Chloro-10H-phenothiazin-10-yl)propyl]-3-ethyl-4-hydroxy-4-methyl-2-oxo-1-oxa-3,8-diazaspiro[4,5]decane, m.p.: 155-156 °C; the hydrochloride decomposes at 220-222 °C;

8-[3-(2-Chloro-10H-phenothiazin-10-yl)propyl]-4-methylene-2-oxo-3-n-propyl-1-oxa-3,8-diazaspiro[4, 5]decane, m.p.: 118-119 °C;

3-Ethyl-4-methylene-2-oxo-8-[3-(2-trifluoromethyl-10H-phenothiazin-10-yl)propyl]-2-oxa-3,8-diazaspiro[4,5]decane, m.p.: 117-119 °C;

3-n-Butyl-4-methylene-2-oxo-8-[3-(2-trifluoromethyl-10H-phenothiazin-10-yl)propyl]-1-oxa-3,8-diazaspiro[4,5]decane, m.p.: 104-105 °C;

4-Methylene-2-oxo-3-tert-butyl-8-[3-(2-trifluoromethyl-10H-phenothiazin-10-yl)propyl]-1-oxa-3,8-diazaspiro[4,5]decane, m.p.: 129-130 °C; and

8-[2-(2-Chloro-10H-phenothiazin-10-yl)ethyl]-3-n-butyl-4-me thylene-2-oxo-1-oxa-3,8-diazaspiro[4,5] decane, m.p.: 108-110 °C.

## Example 5

4-methylene-2-oxo-8-[3-(10H-phenothiazin-10-yl)propyl]-3-phenyl-1-oxa-3,8-diazaspiro[4,5]decane

12.0 g of 4-ethynyl-[3-(10H-phenothiazin-10-yl)propyl]-4-phenylcarbamoyloxypiperidine are refluxed in 120 ml of 0.05 mol/litre methanolic sodium methoxide solution under argon for 3 to 4 hours. After cooling down the sodium methoxide is decomposed by adding aqueous ammonium chloride solution, then the solution is evaporated to the tenth of its original volume under reduced pressure, the residue is diluted with water and extracted with benzene. After washing the benzene phase with water to neutral and drying over anhydrous sodium sulfate the solvent is distilled off and the evaporation residue is recrystallized from ethyl acetate to give the title compound in 85.4% yield, m.p.: 98-99 °C.

Analysis:

calculated for $C_{29}H_{29}N_3O_3S$:

C 72.02; H 6.04; N 8.69; S 6.63%;

found: C 72.12; H 6.19; N 8.48; S 6.70%.

Using the appropriate starting materials the following compound may be prepared in an analogous manner to the process described in the preceding Example.

3-Isopropyl-4-methylene-2-oxo-8-[3-(10H-phenothiazin-10-yl)propyl]-1-oxa-3,8-diazaspiro[4,5]decane, m.p.: 113-114 °C.

Example 6

4-methylene-2-oxo-3-phenyl-8-[3-(2-trifluoromethyl-10H-phenothiazin-10-yl)propyl]-1-oxa-3,8-diazaspiro[4,5]decane

A mixture containing 10.8 g of 4-ethynyl-4-hydroxy-1-[3-(2-trifluoromethyl-10H-phenothiazin-10-yl)propyl]piperidine, 0.12 g of potassium acetate, 3.6 g of phenyl isocyanate and 35 ml of 2-picoline is refluxed under argon while stirring for 4.5 hours. After evaporating the solvent under reduced pressure water is added to the residue and the mixture is extracted with chloroform. The chloroform phase is washed with water, dried over anhydrous sodium sulfate and evaporated under reduced pressure. After recrystallizing the crude product from ethyl acetate under clarifying with activated carbon the title compound is obtained in 74.6% yield, m.p.: 154-155 °C.

Analysis:

calculated for $C_{30}H_{28}F_3N_3O_2S$:

C 65.32; H 5.12; F 10.33; N 7.62; S 5.81%;

found: C 65.40; H 5.31; F 10.12; N 7.57; S 5.88%.

Using the appropriate starting materials the following compounds may be prepared in an analogous manner to the process described in the preceding Example.

8-[3-2-Chloro-10H-phenothiazin-10-yl)propyl]-4-methylene-2-oxo-3-phenyl-1-oxa-3,8-diazaspiro[4,5]decane, m.p.: 145-146 °C;

3-Cyclohexyl-4-methylene-2-oxo-8-[3-(2-trifluoromethyl-10H-phenothiazin-10-yl)propyl]-oxa-3,8-diazaspiro[4,5]decane, m.p.: 175-176 °C.

Example 7

4-hydroxy-3-isopropyl-4-methyl-2-oxo-8-[3-(2-trifluoromethyl-10H-phenothiazin-10-yl)propyl]-1-oxa-3,8-diazaspiro[4,5]decane

A solution containing 5.4 g of 4-acetyl-4-isopropylcarbamoyloxy-1-[3-(2-trifluoromethyl-10-H-phenothiazin-10-yl)propyl]piperidine in 54 ml of 0.55 mol/litre ethanolic sodium ethoxide solution is refluxed under nitrogen for 6 hours. After cooling down and decomposing the reaction mixture with aqueous acetic acid solution. Most of the ethanol is distilled off under reduced pressure. The evaporation residue is diluted with water and extracted with methylene chloride. The organic phase is washed with water to neutral, dried over anhydrous magnesium sulfate, then the solvent is evaporated under reduced pressure. After recrystallizing the crude product from benzene the title compound is obtained in 85.7% yield, m.p.: 187-189 °C.

Analysis:

calculated for $C_{27}H_{32}F_3N_3O_3S$:

C 60.54; H 6.02; F 10.64; N 7.84; S 5.99%;

found: C 60.65; H 6.22; F 10.45; N 7.63; S 6.07%.

On adding ethereal hydrogen chloride solution to the solution of the base in ethanol the hydrochloride is precipitated in crystalline form. It may be filtered and dried to give m.p.: 212-214 °C.

Example 8

3-n-butyl-4-methylene-2-oxo-8-[3-(10H-phenothiazin-10-yl)propyl]-1-oxa-3,8-diazaspiro[4,5]decane

A mixture of 7.6 g of 4-acetyl-4-hydroxy-1-[3-(10H-phenothiazin-10-yl)propyl]piperidine, 1.5 ml of triethylamine and 11.5 ml of n-butyl isocyanate is refluxed under argon for 6 to 7 hours. After cooling down and adding

n-hexane to the reaction mixture the precipitate is filtered off, dissolved in ethyl acetate and led through a silica gel layer. After evaporating the solvent under reduced pressure and recrystallizing the evaporation residue from ethanol the title product is obtained in 67.4% yield, m.p.: 82-83 °C.

Analysis:

calculated for $C_{27}H_{33}N_3O_2S$:

C 69.94; H 7.17; N 9.06; S 6.92%;

found: C 70.17; H 7.28; N 7.28; S 6.77%.

Example 9

8-[3-(2-chloro-10H-phenothiazin-10-yl)propyl]-3-ethyl-4-methylene-2-oxo-1-oxa-3,8-diazaspiro[4,5]decane

9.8 g of 8-[3-(2-chloro-10H-phenothiazin-10-yl)propyl]-3-ethyl-4-hydroxy-4-methyl-2-oxo-1-oxa-3,8-diazaspiro[4,5]decane are boiled with 0.8 g of p-toluenesulfonic acid monohydrate in 150 ml of xylene in a flask equipped with a water-trap, while azeotropically distilling off the water formed in the reaction. After termination of the reaction (2 to 3 hours) the reaction mixture is cooled down, made alkaline by adding aqueous sodium hydroxide solution, the organic phase is separated, washed with water to neutral, dried over anhydrous sodium sulfate and evaporated under reduced pressure. After recrystallization of the evaporation residue from ethyl acetate the title compound is obtained in 87.5% yield, m.p.: 145-146 °C.

Analysis:

calculated for $C_{25}H_{28}ClN_3O_2S$:

C 63.88; H 6.00; Cl 7.54; N 8.94; S 6.82%;

found: C 64.02; H 5.86; Cl 7.37; N 8.83; S 6.60%.

Using the appropriate starting materials the following compounds may be prepared in an analogous manner to the process described in the preceding Example.

8-[3-(2-Chloro-10H-phenothiazin-10-yl)propyl]-4-methylene-2-oxo-3-tert-butyl-1-oxa-3,8-diazaspiro[4, 5]decane, m.p.: 79-81 °C.

3-Isopropyl-4-methylene-2-oxo-8-[3-(2-trifluoromethyl-10H-phenothiazin-10-yl)propyl]-1-oxa-3,8-diazaspiro[4,5]decane, m.p. 129-130 °C.

Example 10

4-methylene-2-oxo-8-[3-(2-trifluoromethyl-10H-phenothiazin-10-yl)propyl]-1,3-dioxa-8-azaspiro[4,5]decane hydrochloride

Into a solution containing 10.0 g of 4-butylcarbamoyloxy-4-ethynyl-1-[3-(2-trifluoromethyl-10H-phenothiazin-10-yl)propyl]piperidine in 50 ml of anhydrous dioxan gaseous dry hydrogen chloride is introduced until saturation at 15 to 20 °C, then the reaction mixture is left to stand overnight. After evaporating the solvent under reduced pressure water is added to the evaporation residue, the precipitate is filtered off, washed with water and dried to obtain the title hydrochloride salt in 88.5% yield, m.p.: 230-233 °C (with decomposition).

The base can be liberated from its hydrochloride by adding sodium hydrogen carbonate.

Analysis of the base:

calculated for $C_{24}H_{23}F_3N_2O_3S$:

C 60.49; H 4.86; F 11.96; N 5.88; S 6.73%;

found: C 60.60; H 4.99; F 11.78; N 6.03; S 6.70%.

Example 11

8-methyl-4-methylene-2-oxo-8-[3-(10H-phenothiazin-10-yl)propyl]-3-propyl-1-oxa-3,8-diazaspiro[4,5]decan-8-ium iodide

6.0 g of 4-methylene-2-oxo-8-[3-(10H-phenothiazin-10-yl)propyl]-3-propyl-1-oxa-3,8-diazaspiro[4,5]decane are boiled under reflux with 2.9 g of methyl iodide in 60 ml of methyl isobutyl ketone for 2 hours. After cooling down the crystalline precipitate is filtered off and washed with diisopropyl ether previously cooled to 0°C. After drying the title quaternary ammonium salt is obtained in 98.0% yield, m.p.: 192-193 °C.

The following compound may be prepared in an analogous manner to the process described in the preceding Example using the appropriate precursors: 8-Allyl-3-butyl-4-methylene-2-oxo-8-[3-(10H-phenothiazin-10-yl)propyl]-1-oxa-3,8-diazaspiro[4,5]decan-8-ium bromide, m.p.: 200-202 °C.

Example 12

4-hydroxy-4-methyl-2-oxo-3-propyl-8-[3-(2-trifluoromethyl-10H-phenothiazin-10-yl)propyl]-1-oxa-3,8-diazaspiro[4,5]decane

4.8 g of 4-methylene-2-oxo-8[3-(2-trifluoromethyl-10H-phenothiazin-10-yl)propyl]-1,3-dioxa-8-azaspiro[4,5]decane are stirred with 50 ml of n-propylamine overnight, then the excess amine is distilled off under reduced pressure. After recrystallizing the evaporation residue from benzene the title compound is obtained in 89.8% yield, m.p.: 170.5-172 °C.

Analysis:

calculated for $C_{27}H_{32}F_3N_3O_3S$:

C 60.54; H 6.02; F 10.64; N 7.84; S 5.99%;

found: C 60.47; H 6.11; F 10.71; N 7.89; S 6.15%.

The hydrochloride of the title compound is prepared by treating an ethanolic solution of the base with ethereal hydrogen chloride solution, m.p.: 129-133 °C.

Using the appropriate starting materials the following compound may be prepared in an analogous manner to the process described in the preceding Example:

4-Hydroxy-4-methyl-2-oxo-8-[3-(2-trifluoromethyl-10H-phenothiazin-10-yl)propyl]-1-oxa-3,8-diazaspiro[4,5]decane, m.p.: 102-103 °C.

Example 13

3,4-dimethyl-4-hydroxy-2-oxo-8-[3-(2-trifluoromethyl-10H-phenothiazin-10-yl)propyl]-1-oxa-3,8-diazaspiro[4,5]decane

After adding a solution of 0.4 g of methylamine in 10 20 ml of xylene (previously cooled to 0 °C) to the solution of 4.8 g of 4-methylene-2-oxo-8-[3-(2-trifluoromethyl-10H-phenothiazin-10-yl)propyl]-1,3-dioxa-8-azaspiro[4,5]decane in ml of xylene while stirring, the reaction mixture is heated at 70 to 80 °C for 60 minutes, then the solvent is distilled off under reduced pressure. After recrystallizing the evaporation residue from a mixture of ethanol and n-hexane the title product is obtained in 81.5% yield, m.p.: 164-165 °C.

Analysis:

calculated for $C_{25}H_{28}F_3N_3O_3S$:

C 59.15; H 5,56; F 11.23; N 8.28; S 6.32%;

found: C 59.32; H 5.39; F 11.14; N 8.43; S 6.50%.

The hydrochloride of the title base melts at 194-197 °C with decomposition.

Example 14

3-ethyl-4-methylene-2-oxo-8-[3-(10H-phenothiazin-10-yl)propyl]-1-oxa-3,8-diazaspiro[4,5]decane

0.6 g of sodium hydride (60 % oily dispersion) is added to a solution of 3.0 g of phenothiazine in 20 ml of anhydrous dimethylformamide under argon, then the reaction mixture is stirred at 50 to 60 °C for 2 hours. Thereafter, 3.9 g of 8-(3-chloropropyl)-3-ethyl-4-methylene-2-oxo-1-oxa-3,8-diazaspiro[4,5]decane dissolved in 20 ml of dimethylformamide are dropwise added and the mixture is stirred at 40 to 50 °C for additional 6 to 7 hours. After cooling down saturated ammonium chloride solution is added to the reaction mixture under argon and the solvent is evaporated under reduced pressure. After taking up the evaporation residue in benzene and washing the benzene solution with water the solution is dried over anhydrous sodium sulfate and then evaporated under reduced pressure. The residue is recrystallized from ethanol to give the title product in 57.6% yield, m.p.: 122-123 °C.

Analysis:

calculated for $C_{25}H_{29}N_3O_2$:

C 68.93; H 6.71; N 9.65; S 7.36%;

found: C 62.08; H 6.77; N 9.78; S 7,23%.

Non-limiting Examples of pharmaceutical compositions that may be prepared using the compounds according to the present invention include:

(a) Preparation of tablets with a weight of 100 mg containing 10 mg of active ingredient each.

50.0 g of active ingredient are mixed together with 285.0 g of lactose, 100.0 g of potato starch, 2.5 g of sodium dodecyl sulfate, 5.0 g of polyvinylpyrrolidone (Kollidon-K 90$^R$), 50.0 g of microcrystalline cellulose (Avicel$^R$) and 7.5 g of vegetable oil (sterotex$^R$) and, after wet granulation, the product obtained is compressed to tablets weighing 100 mg each. Each of the tablets contains 10 mg of the active ingredient.

(b) Preparation of dragées with a weight of 125 mg containing 10 mg of active ingredient each

After coating the tablets prepared as described above in a known manner with a layer consisting of sugar and talc, the dragées so obtained are polished with a mixture of bees wax and carnauba wax.

(c) Preparation of capsules containing 20 mg of active ingredient each

40.0 g of active ingredient, 12.0 g of sodium lauryl sulfate, 102.0 g of lactose, 102.0 g of potato starch, 2.4 g of magnesium stearate and 1.6 g of colloidal silicon dioxide are thoroughly mixed together and the mixture obtained is filled into hard gelatine capsules containing 20 mg of active ingredient each.

## Claims

### Claims for the following Contracting States : AT, BE, CH, DE, DK, FR, GB, IT, LI, LU, NL, SE

1. A compound of formula (I),

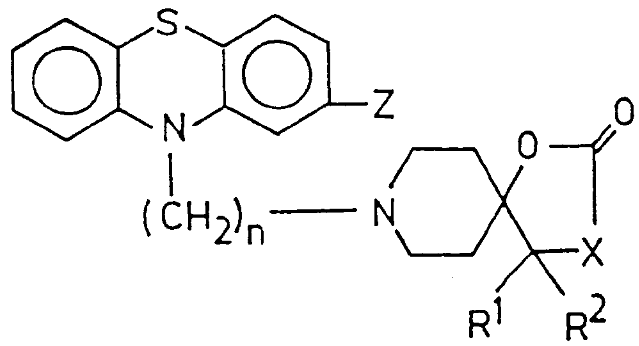

(I)

wherein

X represents an oxygen atom or a NR group, wherein

R represents a hydrogen atom or a $C_{1-12}$ alkyl or $C_{3-6}$cycloalkyl group, or a carbocyclic $C_{6-10}$aryl or carbocylic $C_{6-10}$aryl-$C_{1-4}$alkyl group optionally substituted on the aromatic ring by one or more halogen atoms, $C_{1-4}$ alkyl or $C_{1-4}$alkoxy groups;

$R^1$ and $R^2$ together represent a methylene group or when X is a >NR group, one of $R^1$ and $R^2$ may additionally represent a hydroxyl group and the other represent a methyl group;

Z represents a hydrogen or halogen atom or a trihalomethyl or $C_{2-4}$alkanoyl group; and

n is 2 or 3;

and optical isomers and mixtures thereof and all acid addition and quarternary ammonium salts thereof.

2. A compound of formula (I) as defined in Claim 1 selected from the group comprising:
8-[3-(2-chloro-10H-phenothiazin-10-yl)propyl] 3-methyl-4-methylene-2-oxo-1-oxa-3,8-diazaspiro [4,5] decane,
3-methyl-4-methylene-2-oxo-8-3-(2-trifluoromethyl-10H-phenothiazin-10-yl)propyl]-1-oxa-3,8-diazaspiro [4,5] decane,
3-ethyl-4-methylene-2-oxo-8-[3-(2-trifluoromethyl-10H-phenothiazin-10-yl)propyl]-1-oxa-3,8-diazaspiro, [4,5] decane,
8-[3-(2-chloro-10H-phenothiazin-10-yl)propyl]-3-ethyl-4-methylene-2-oxo-1-oxa-3,8-diazaspiro [4,5] decane,
8-[3(2-chloro-10H-phenothiazin-10-yl)propyl]-3,4-dimethyl-4-hydroxy-2-oxo-1-oxa-3,8-diazaspiro [4,5] decane,
3,4-dimethyl-4-hydroxy-2-oxo-8-[3-(2-trifluoromethyl-10H-phenothiazin-10-yl)propyl]-1-oxa-3,8-diazaspiro [4,5] decane,
4-methylene-2-oxo-8-[3-(2-trifluoromethyl-10H-phenothiazin-10-yl)propyl]-1,3-dioxa-8-diazaspiro [4,5] decane,
and optical isomers and mixtures thereof and all acid addition and quaternary ammonium salts thereof.

3. A pharmaceutical composition which comprises a compound of formula (I) as defined in claim 1 or a pharmaceutically active acid addition or quaternary ammonium salt thereof in admixture with a pharmaceutically acceptable carrier and/or additive.

4. A process for the preparation of a compound of formula (I) as defined in Claim 1 which comprises
a) for the preparation of a compound of formula (I) wherein X is a group NR, reacting a 2-oxo-3,8-diazaspiro [4,5] decane derivative of of formula (II),

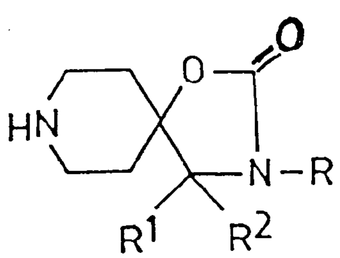

(II)

wherein R,$R^1$ and $R^2$ are as defined in Claim 1 above, with a phenothiazine derivative of the formula (III),

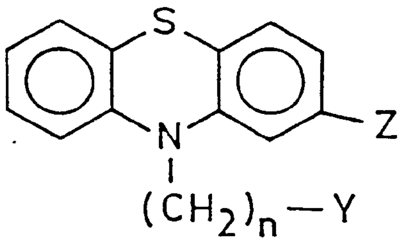

(III)

wherein n and Z are as defined in Claim 1 and Y represents a halogen atom or a $C_{1-4}$alkylsulfonyloxy or arylsulfonloxy group;

or

b) reacting a 2-oxo-3,8-diazaspiro [4,5] decane derivative of formula (IV),

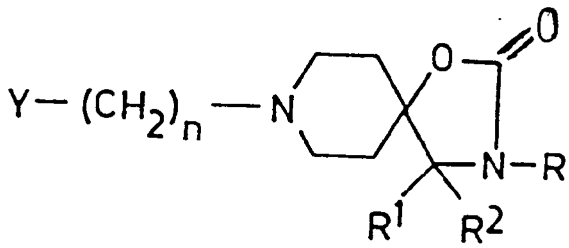

(IV)

wherein R, n, $R^1$, $R^2$ and Y are as defined above with a phenothiazine derivative of formula (V),

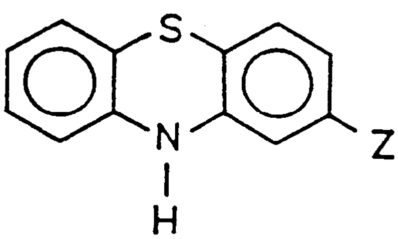

(V)

wherein Z is as defined above;

or

c) for the preparation of compounds of formula (I) wherein $R_1$ and $R_2$ together are a methylene group, reacting a compound of formula (VI)

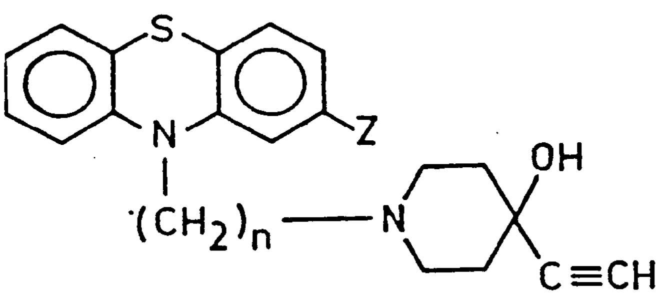

(VI)

wherein n and Z are as defined above, with an isocyanate of formula R-NCO, wherein R is as defined above, to obtain a 4-carbamoyloxy-4-ethynylpiperdine derivative of the formula (VII),

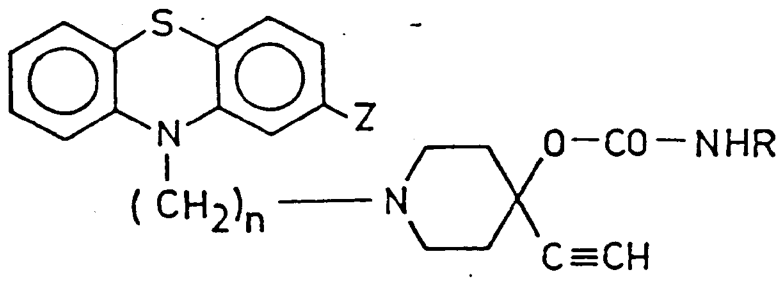

(VII)

wherein R, n and Z are defined above followed by either:

α) for compounds wherein X is a oxygen atom, cyclizing in an acidic medium the compound of formula (VII) as prepared and defined above and reacting the resulting salt formula (VIII),

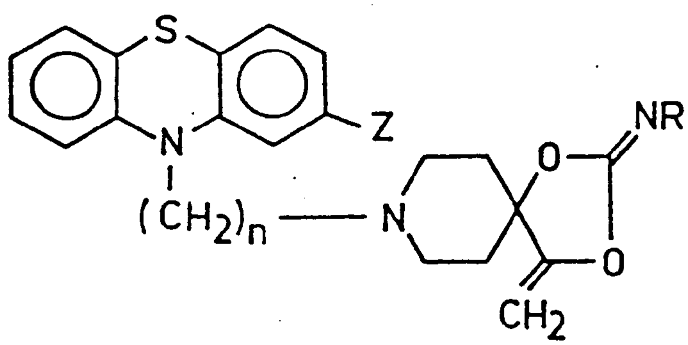

(VIII)

wherein R, n and Z are as defined above with water;

or

β) for compounds wherein X is an NR group, cyclizing in a basic medium the compound of formula (VII) as prepared and defined above;

or

d) for the preparation of compounds of formula (I) as defined in process (c) (α) above, cyclizing in an acidic medium a 4-carbamoyloxy-4-ethynylpiperdine derivative of formula (VII) as defined above and reacting the obtained salt of formula (VIII) as defined above with water;

or

e) for the preparation of compounds of formula (I) as defined in process (c) (β) above, cyclizing in a basic medium a 4-carbamoyloxy-4-ethynylpiperidine derivative of formula (VII) as defined above;

or

f) for the preparation of compounds of formula (I) wherein X is a >NR group and one of $R^1$ and $R^2$ is hydroxyl group and the other is a methyl group, reacting a 4-acetyl-4-hydroxypiperidine derivative of formula (X),

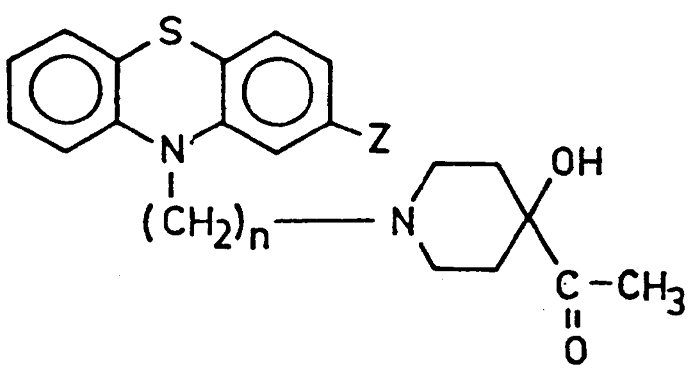

(X)

wherein n and Z are as defined above, with an isocyanate of formula R-NCO, wherein R is as defined above, to obtain a 4-acetyl-4-carbamoyloxypiperidine derivative of the formula (IX),

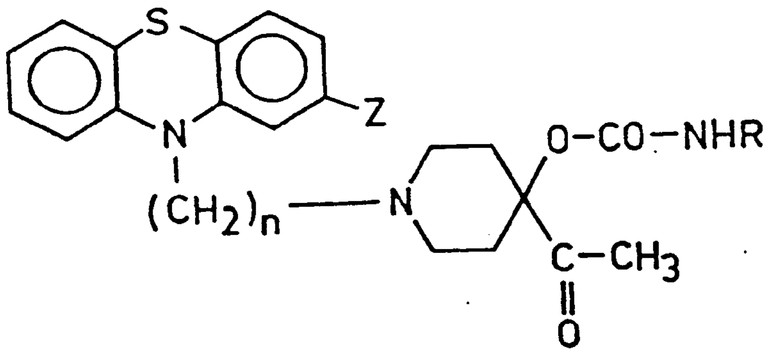

(IX)

wherein R, n and Z are as defined above, which is then cyclized;
or

g) for the preparation of compounds of formula (I) wherein X is a >NR group and one of $R^1$ and $R^2$ is a hydroxyl group and the other is a methyl group, cyclizing a 4-acetyl-4-carbamoyloxypiperdine derivative of formula (IX) of as defined above;

followed if desired or necessary,
by reacting a compound of the formula (I) as prepared above, wherein X is an oxygen atom and $R^1$ and $R^2$ together are a methylene group and n and Z are as defined above, with an amine of formula $R\text{-}NH_2$, wherein R is as defined above, in order to prepare a compound of the formula (I), wherein X is a NR group and one of $R^1$ and $R^2$ is a hydroxyl group and the other is a methyl group;
and/or if desired or necessary,
transforming a functional group of a compound of formula (I) as prepared above into another compound of formula (I) as defined in Claim 1;
and/or if desired or necessary,
reacting a compound of formula (I) as prepared above with an acid to obtain the acid addition salt thereof and/or reacting a base with a salt of a compound of formula (I) as prepared above to liberate the free basic form thereof and/or converting a thus prepared compound of formula (I) into its quaternary ammonium salt.

5. A process as claimed in Claim 4, wherein Y is either a bromine or chlorine atom or a p-toluenesulfonyloxy group.

6. A process as claimed in Claim 4 process (c) (β) wherein the reaction is carried out as a single step without isolating the compound of the formula (VII).

7. A process as claimed in Claim 4 process (d) in which the cyclization of the 4-carbamoyloxy-4-ethynylpiperdine derivative of the formula (VII) is carried out in dioxan and in the presence of hydrogen chloride.

8. A process as claimed Claim 4 process (e) in which the cyclization of the 4-carbamoyloxy-4-ethynylpipridine derivative of the formula (VII) is carried out in methanol and in the presence of sodium methoxide.

9. The use of a compound of formula (I) as defined in Claim 1 in the manufacture of a medicament for the treatment of psychiatric and allergic diseases in mammals.

10. A process for the preparation of a pharmaceutical composition as defined in Claim 3 in which a compound of formula (I) is defined in Claim 1 or a pharmaceutically acid addition or quaternary ammonium salt thereof is admixed with a pharmaceutically acceptable carrier and/or additive.

**Claims for the following Contracting States : ES, GR**

1. A process for the preparation of a compound of formula (I)

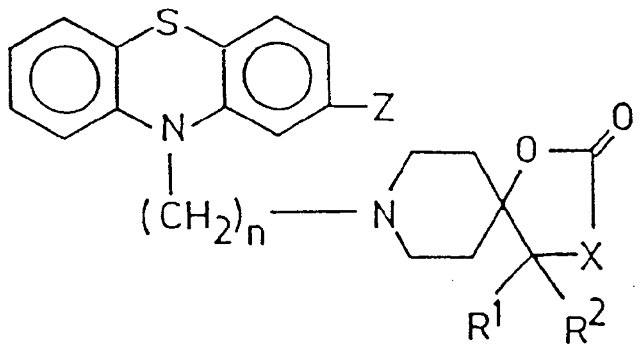

(I)

wherein

X represents an oxygen atom or a NR group, wherein

R represents a hydrogen atom or a $C_{1-12}$ alkyl or $C_{3-6}$cycloalkyl group, or a carbocyclic $C_{6-10}$aryl or carbocylic $C_{6-10}$aryl-$C_{1-4}$alkyl group optionally substituted on the aromatic ring by one or more halogen atoms, $C_{1-4}$ alkyl or $C_{1-4}$alkoxy groups;

$R^1$ and $R^2$ together represent a methylene group or when X is a >NR group, one of $R^1$ and $R^2$ may additionally represent a hydroxyl group and the other represent a methyl group;

Z represents a hydrogen or halogen atom or a trihalomethyl or $C_{2-4}$alkanoyl group; and

n is 2 or 3;

and optical isomers and mixtures thereof and all acid addition and quarternary ammonium salts thereof, which comprises

a) for the preparation of a compound of formula (I) wherein X is a group NR, reacting a 2-oxo-3,8-diazaspiro [4,5] decane derivative of of formula (II),

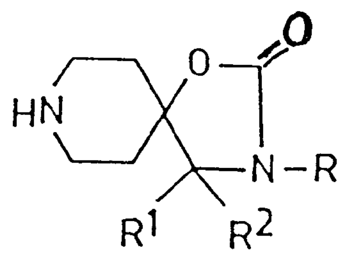

(II)

wherein R,$R^1$ and $R^2$ are as defined above, with a phenothiazine derivative of the formula (III),

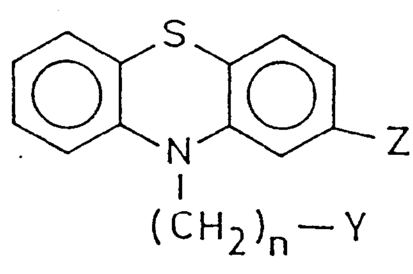

(III)

wherein n and Z are as defined above and Y represents a halogen atom or a $C_{1-4}$alkylsulfonyloxy or arylsulfonloxy group;

or

b) reacting a 2-oxo-3,8-diazaspiro [4,5] decane derivative of formula (IV),

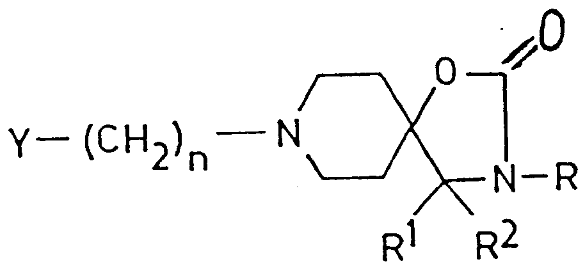

(IV)

wherein R, n, $R^1$, $R^2$ and Y are as defined above with a phenothiazine derivative of formula (V),

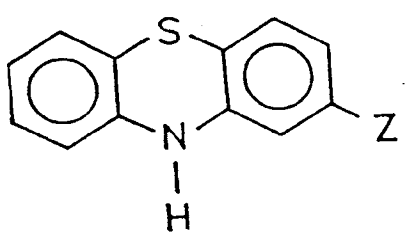

(V)

wherein Z is as defined above;
or
c) for the preparation of compounds of formula (I) wherein $R_1$ and $R_2$ together are a methylene group, reacting a compound of formula (VI)

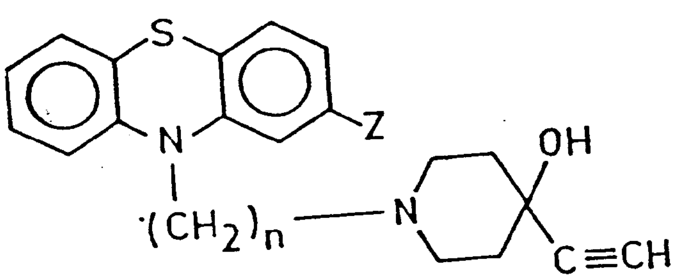

(VI)

wherein n and Z are as defined above, with an isocyanate of formula R-NCO, wherein R is as defined above, to obtain a 4-carbamoyloxy-4-ethynylpiperdine derivative of the formula (VII),

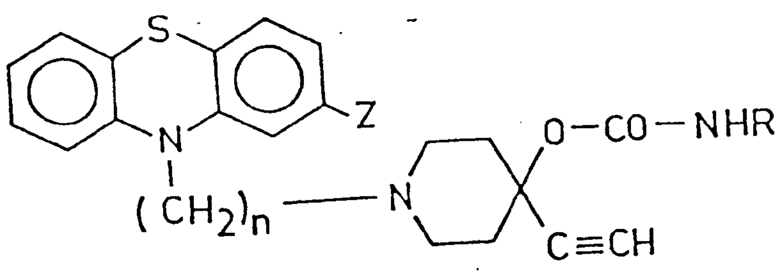

(VII)

wherein R, n and Z are defined above followed by either:
α) for compounds wherein X is a oxygen atom, cyclizing in an acidic medium the compound of formula (VII) as prepared and defined above and reacting the resulting salt formula (VIII),

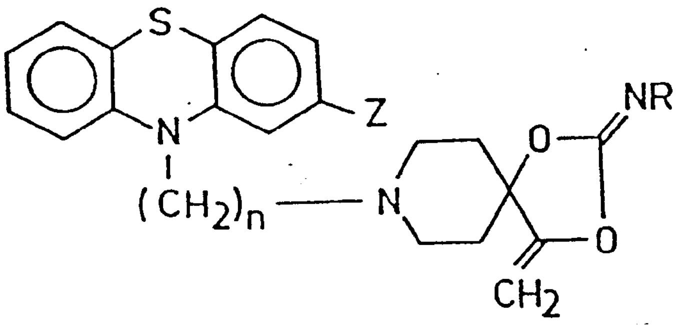

(VIII)

wherein R, n and Z are as defined above with water;

or

β) for compounds wherein X is an NR group, cyclizing in a basic medium the compound of formula (VII) as prepared and defined above;

or

d) for the preparation of compounds of formula (I) as defined in process (c) (α) above, cyclizing in an acidic medium a 4-carbamoyloxy-4-ethynylpiperdine derivative of formula (VII) as defined above and reacting the obtained salt of formula (VIII) as defined above with water;

or

e) for the preparation of compounds of formula (I) as defined in process (c) (β) above, cyclizing in a basic medium a 4-carbamoyloxy-4-ethynylpiperidine derivative of formula (VII) as defined above;

or

f) for the preparation of compounds of formula (I) wherein X is a >NR group and one of $R^1$ and $R^2$ is hydroxyl group and the other is a methyl group, reacting a 4-acetyl-4-hydroxypiperidine derivative of formula (X),

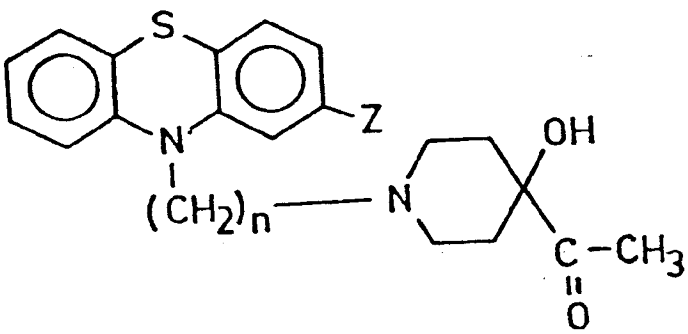

(X)

wherein n and Z are as defined above, with an isocyanate of formula R-NCO, wherein R is as defined above, to obtain a 4-acetyl-4-carbamoyloxypiperidine derivative of the formula (IX),

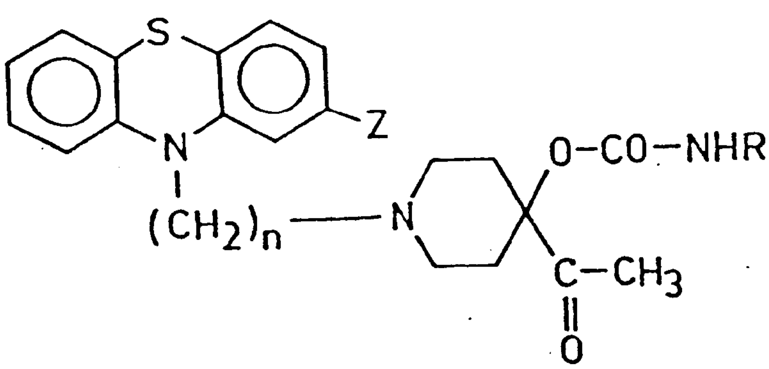

(IX)

wherein R, n and Z are as defined above,

which is then cyclized;

or

g) for the preparation of compounds of formula (I) wherein X is a >NR group and one of $R^1$ and $R^2$ is a hydroxyl group and the other is a methyl group, cyclizing a 4-acetyl-4-carbamoyloxypiperdine derivative of formula (IX) of as defined above;

followed if desired or necessary,

by reacting a compound of the formula (I) as prepared above, wherein X is an oxygen atom and $R^1$ and $R^2$ together are a methylene group and n and Z are as defined above, with an amine of formula $R\text{-}NH_2$, wherein R is as defined above, in order to prepare a compound of-the formula (I), wherein X is a NR group and one of $R^1$ and $R^2$ is a hydroxyl group and the other is a methyl group;

and/or if desired or necessary,

transforming a functional group of a compound of formula (I) as prepared above into another compound of formula (I) as defined above;

and/or if desired or necessary,

reacting a compound of formula (I) as prepared above with an acid to obtain the acid addition salt thereof and/or reacting a base with a salt of a compound of formula (I) as prepared above to liberate the free basic form thereof and/or converting a thus prepared compound of formula (I) into its quaternary ammonium salt.

**2.** A process as claimed in Claim 1, wherein Y is either a bromine or chlorine atom or a p-toluenesulfonyloxy group.

**3.** A process as claimed in Claim 1 process (c) (β) wherein the reaction is carried out as a single step without isolating the compound of the formula (VII).

**4.** A process as claimed in Claim 1 process (d) in which the cyclization of the 4-carbamoyloxy-4-ethynylpiperdine derivative of the formula (VII) is carried out in dioxan and in the presence of hydrogen chloride.

**5.** A process as claimed Claim 1 process (e) in which the cyclization of the 4-carbamoyloxy-4-ethynylpipridine derivative of the formula (VII) is carried out in methanol and in the presence of sodium methoxide.

**6.** A process as claimed in Claim 1 for the preparation of a compound of formula (I) as defined in Claim 1 selected from the group comprising:

8-[3-(2-chloro-10H-phenothiazin-10-yl)propyl] 3-methyl-4-methylene-2-oxo-1-oxa-3,8-diazaspiro [4,5] decane,

3-methyl-4-methylene-2-oxo-8-3-(2-trifluoromethyl-10H-phenothiazin-10-yl)propyl]-1-oxa-3,8-diazaspiro [4,5] decane,

3-ethyl-4-methylene-2-oxo-8-[3-(2-trifluoromethyl-10H-phenothiazin-10-yl)propyl]-1-oxa-3,8-diazaspiro, [4,5] decane,

8-[3-(2-chloro-10H-phenothiazin-10-yl)propyl]-3-ethyl-4-methylene-2-oxo-1-oxa-3,8-diazaspiro [4,5] decane, 8-[3(2-chloro-10H-phenothiazin-10-yl)propyl]-3,4-dimethyl-4-hydroxy-2-oxo-1-oxa-3,8-diazaspiro [4,5] decane,

3,4-dimethyl-4-hydroxy-2-oxo-8-[3-(2-trifluoromethyl-10H-phenothiazin-10-yl)propyl]-1-oxa-3,8-diazaspiro [4,5] decane,

4-methylene-2-oxo-8-[3-(2-trifluoromethyl-10H-phenothiazin-10-yl)propyl]1,3-dioxa-8-diazaspiro [4,5] decane,

and optical isomers and mixtures thereof and all acid addition and quaternary ammonium salts thereof

**7.** The use of a compound of formula (I) as defined in Claim 1 in the manufacture of a medicament for the treatment of psychiatric and allergic diseases in mammals.

**8.** A process for the preparation of a pharmaceutical composition which comprises as active ingredient a compound of formula (I) as defined in Claim 1 or a pharmaceutically acid addition or quaternary ammonium salt thereof, wherein the active ingredient is admixed with a pharmaceutically acceptable carrier and/or additive.

**Patentansprüche**

**Patentansprüche für folgende Vertragsstaaten : AT, BE, CH, DE, DK, FR, GB, IT, LI, LU, NL, SE**

1. Verbindung der Formel (I)

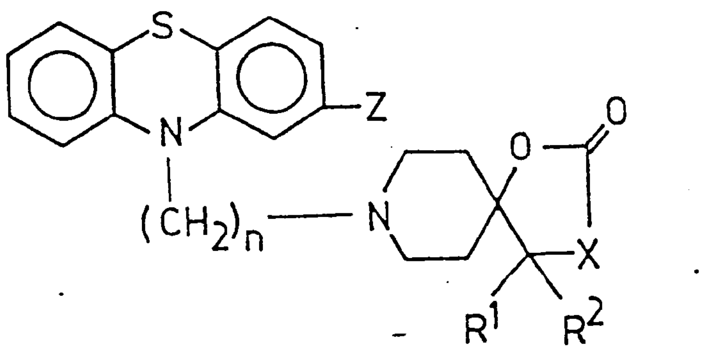

(I)

worin
X ein Sauerstoffatom oder eine NR-Gruppe bedeutet,
worin
R ein Wasserstoffatom oder eine $C_{1-12}$-Alkyl- oder $C_{3-6}$-Cycloalkylgruppe, oder eine carbozyklische $C_{6-10}$-Aryl- oder carbozyklische $C_{6-10}$-Aryl-$C_{1-4}$-alkylgruppe ist, gegebenenfalls an dem aromatischen Ring durch ein oder mehrere Halogenatome, $C_{1-4}$-Alkyl- oder $C_{1-4}$-Alkoxygruppen substituiert;
worin
$R^1$ und $R^2$ zusammen eine Methylengruppe sind, oder, wenn X eine >NR-Gruppe ist, kann eines von $R^1$ und $R^2$ zusätzlich eine Hydroxylgruppe und das andere eine Methylgruppe bedeuten;
worin
Z ein Wasserstoff- oder Halogenatom oder eine Trihalomethyl- oder $C_{2-4}$-Alkanoylgruppe ist; und
worin
n 2 oder 3 ist;
und optische Isomere und Mischungen davon und alle Säureadditions- und quartären Ammoniumsalze davon.

2. Verbindung der Formel (I) nach Anspruch 1, ausgewählt aus der Gruppe, umfassend:
8-[3-(2-Chloro-10H-phenothiazin-10-yl)propyl]-3-methyl-4-methylen-2-oxo-1-oxa-3,8-diazaspiro[4,5]decan,
3-Methyl-4-methylen-2-oxo-8-[3-(2-trifluoromethyl-10H-phenothiazin-10-yl)propyl]-1-oxa-3,8-diazaspiro[4,5]decan,
3-Ethyl-4-methylen-2-oxo-8-[3-(2-trifluoromethyl-10H-phenothiazin-10-yl)propyl]-1-oxa-3,8-diazaspiro[4,5]decan,
8-[3-(2-Chloro-10H-phenothiazin-10-yl)propyl]-3-ethyl-4-methylen-2-oxo-1-oxa-3,8-diazaspiro[4,5]decan,
8-[3-(2-Chloro-10H-phenothiazin-10-yl)propyl]-3,4-dimethyl-4-hydroxy-2-oxo-1-oxa-3,8-diazaspiro[4,5]decan,
3,4-Dimethyl-4-hydroxy-2-oxo-8-[3-(2-trifluoromethyl-10H-phenothiazin-10-yl)propyl]-1-oxa-3,8-diazaspiro[4,5]decan,
4-Methylen-2-oxo-8-[3-(2-trifluoromethyl-10H-phenothiazin-10-yl)propyl]-1,3-dioxa-8-diazaspiro[4,5]decan,
und optische Isomere und Mischungen davon und alle Säureadditions- und quartären Ammoniumsalze davon.

3. Pharmazeutische Zusammensetzung, umfassend eine Verbindung der Formel (I) nach Anspruch 1 oder ein pharmazeutisch aktives Säureadditionssalz oder quartäres Ammoniumsalz davon in Zumischung mit einem pharmazeutisch akzeptablen Träger und/oder Additiv.

4. Verfahren zur Herstellung einer Verbindung der Formel (I) nach Anspruch 1, umfassend
(a) für die Herstellung einer Verbindung der Formel (I), worin X eine Gruppe NR ist, Reaktion eines 2-Oxo-3,8-diazaspiro[4,5]decanderivates der Formel (II)

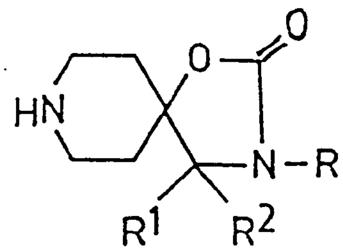

(II)

worin R, $R^1$ und $R^2$ wie oben in Anspruch 1 definiert sind, mit einem Phenothiazinderivat der Formel (III)

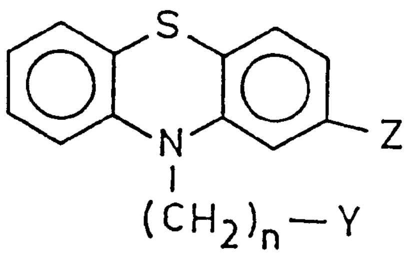

(III)

worin n und Z wie in Anspruch 1 definiert sind und
worin Y ein Halogenatom oder eine $C_{1-4}$-Alkylsulfonyloxy- oder Arylsulfonyloxygruppe ist;
oder
(b) Reaktion eines 2-Oxo-3,8-diazaspiro[4,5]decanderiviates der Formel (IV)

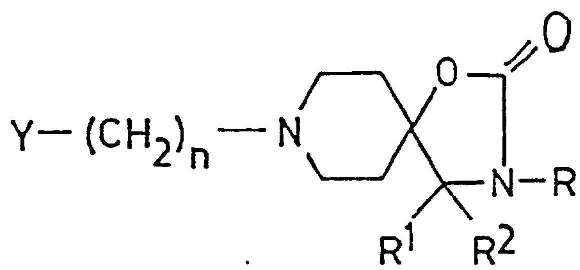

(IV)

worin R, n, $R^1$, $R^2$ und Y wie oben definiert sind, mit einem Phenothiazinderiviat der Formel (V)

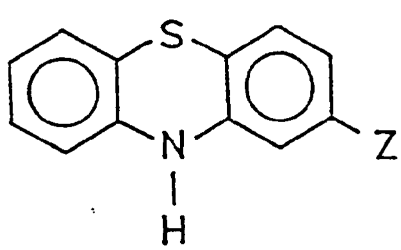

(V)

worin Z wie oben definiert ist;
oder
(c) für die Herstellung von Verbindungen der Formel (I), worin $R_1$ und $R_2$ zusammen eine Methylengruppe sind, Reaktion einer Verbindung der Formel (VI)

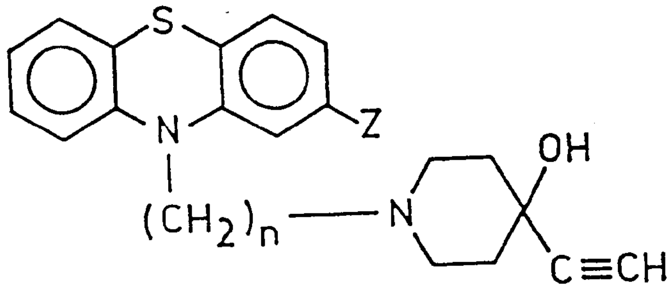

(VI)

wobei n und Z wie oben definiert sind, mit einem Isocyanat der Formel R-NCO, worin R wie oben definiert ist, unter Erhalt eines 4-Carbamoyloxy-4-ethynylpiperidinderivates der Formel (VII)

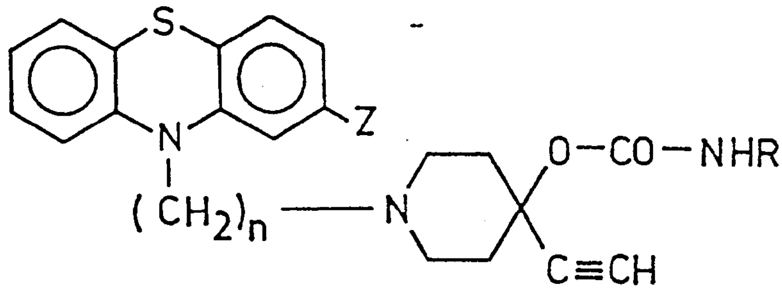

(VII)

worin R, n und Z wie oben definiert sind, gefolgt von einem der folgenden Schritte:
α) für Verbindungen, worin X ein Sauerstoffatom ist, Zyklisierung der Verbindung der Formel (VII), wie oben hergestellt und definiert, in einem sauren Medium und Reaktion des resultierenden Salzes der Formel (VIII)

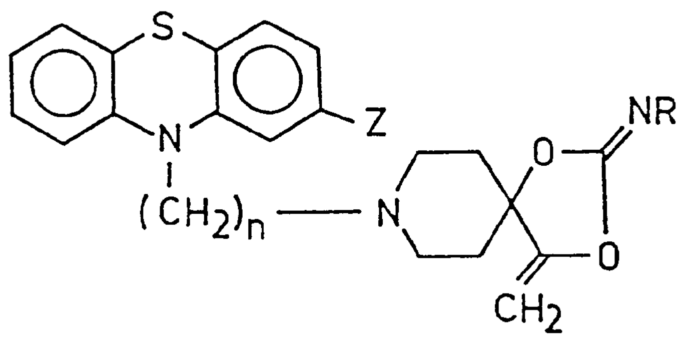

(VIII)

worin R, n und Z wie oben definiert sind, mit Wasser;
oder
β) für Verbindungen, worin X eine NR-Gruppe ist, Zyklisierung der Verbindung der Formel (VII), wie oben hergestellt und definiert, in einem basischen Medium;
oder
d) für die Herstellung von Verbindungen der Formel (I), wie im obigen Verfahren (c) (α) definiert, Zyklisierung eines 4-Carbamoyloxy-4-ethynylpiperidinderivates der Formel (VII) wie oben definiert in einem sauren Medium und Reaktion des erhaltenen Salzes der Formel (VIII) wie oben definiert mit Wasser;
oder
e) für die Herstellung von Verbindungen der Formel (I), wie im obigen Verfahren (c) (β) definiert, Zyklisieren eines 4-Carbamoyloxy-4-ethynylpiperidinderivates der Formel (VII) wie oben definiert in einem basischen Medium;
oder
f) für die Herstellung von Verbindungen der Formel (I), worin X eine >NR-Gruppe ist, und worin eines

von $R^1$ und $R^2$ eine Hydroxylgruppe und das andere eine Methylgruppe ist, Reaktion eines 4-Acetyl-4-hydroxypiperidinderivates der Formel (X)

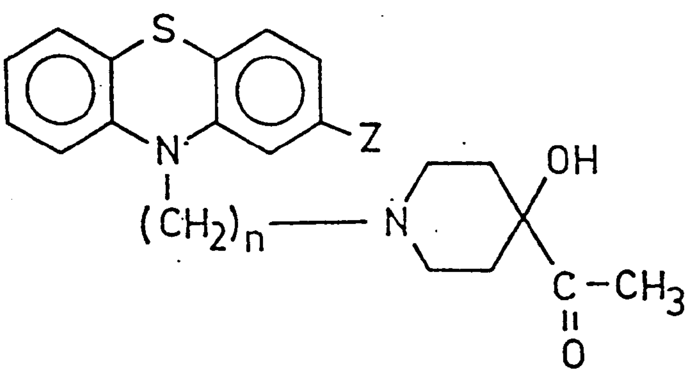

(X)

worin n und Z wie oben definiert sind, mit einem Isocyanat der Formel R-NCO, worin R wie oben definiert ist, unter Erhalt eines 4-Acetyl-4-carbamoyloxypiperidinderviates der Formel (IX)

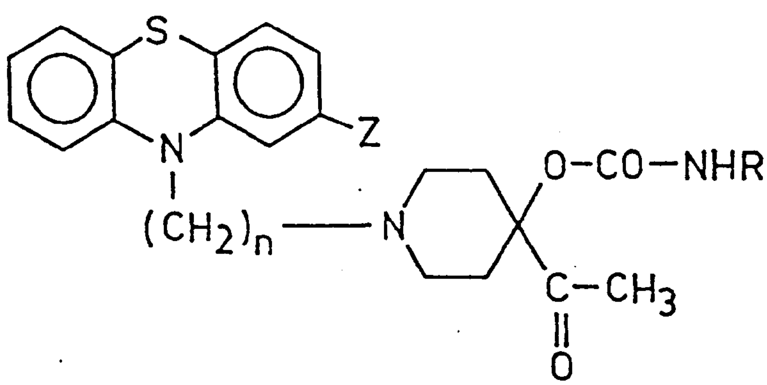

(IX)

worin R, n und Z wie oben definiert sind, das dann zyklisiert wird;
oder
g) für die Herstellung von Verbindungen der Formel (I), worin X eine >NR-Gruppe ist und worin eines von $R^1$ und $R^2$ eine Hydroxylgruppe und das andere eine Methylgruppe ist, Zyklisieren eines 4-Acetyl-4-carbamoyloxypiperidinderivates der Formel (IX) wie oben definiert;
falls erwünscht oder notwendig,
mit anschließender Reaktion einer Verbindung der Formel (I), wie oben hergestellt, worin X ein Sauerstoffatom ist und worin $R^1$ und $R^2$ zusammen eine Methylengruppe sind und worin n und Z wie oben definiert sind, mit einem Amin der Formel $R\text{-}NH_2$, worin R wie oben definiert ist, zur Herstellung einer Verbindung der Formel (I), worin X eine NR-Gruppe ist und worin eines von $R^1$ und $R^2$ eine Hydroxylgruppe und das andere eine Methylgruppe ist;
und/oder falls gewünscht oder erforderlich, Umwandlung einer funktionellen Gruppe einer Verbindung der Formel (I), wie oben hergestellt, in eine andere Verbindung der Formel (I) wie in Anspruch 1 definiert;
und/oder falls erwünscht oder erforderlich, Reaktion einer Verbindung der Formel (I), wie oben hergestellt, mit einer Säure, unter Erhalt des Säureadditionssalzes davon und/oder Reaktion einer Base mit einem Salz einer Verbindung der Formel (I) wie oben hergestellt, zur Freisetzung der freien basischen Form davon und/oder Umwandlung einer somit hergestellten Verbindung der Formel (I) in ihr quartäres Ammoniumsalz.

5. Verfahren nach Anspruch 4, dadurch **gekennzeichnet**, daß Y entweder ein Brom- oder Chloratom oder eine p-Toluolsulfonyloxygruppe ist.

6. Verfahren nach Anspruch 4, Verfahren (c) (β), dadurch **gekennzeichnet** , daß die Reaktion als ein Einzelschritt ohne Isolierung der Verbindung der Formel (VII) durchgeführt wird.

7. Verfahren nach Anspruch 4, Verfahren (d), dadurch **gekennzeichnet**, daß die Zyklisierung des 4-Carbamoyloxy-4-ethynylpiperidinderivates der Formel (VII) in Dioxan und in der Gegenwart von Chlorwasserstoff durchgeführt wird.

8. Verfahren nach Anspruch 4, Verfahren (e), dadurch **gekennzeichnet**, daß die Zyklisierung des 4-Carbamoyloxy-4-ethynylpiperidinderivates der Formel (VII) in Methanol und in der Gegenwart von Natriummethoxid durchgeführt wird.

9. Verwendung einer Verbindung der Formel (I) nach Anspruch 1 für die Herstellung eines Medikamentes für die Behandlung von psychiatrischen und allergischen Erkrankungen in Säugern.

10. Verfahren zur Herstellung einer pharmazeutischen Zusammensetzung nach Anspruch 3, worin eine Verbindung der Formel (I) wie in Anspruch 1 definiert, oder ein pharmazeutisch akzeptables Säureadditions- oder quartäres Ammoniumsalz davon mit einem pharmazeutisch akzeptablen Träger und/oder Additiv vermischt wird.

**Patentansprüche für folgende Vertragsstaaten : ES, GR**

1. Verfahren zur Herstellung einer Verbindung der Formel (I)

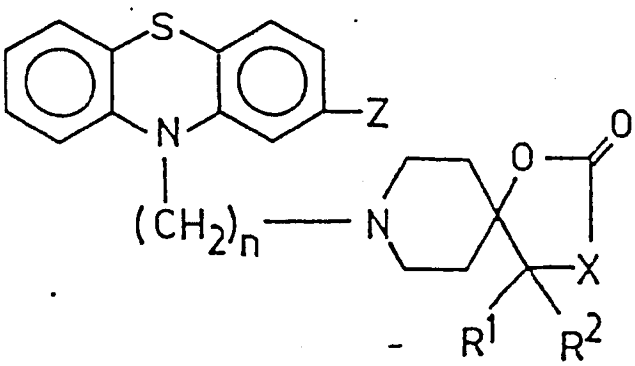

(I)

worin

X ein Sauerstoffatom oder eine NR-Gruppe bedeutet,
worin
R ein Wasserstoffatom oder eine $C_{1-12}$-Alkyl- oder $C_{3-6}$-Cycloalkylgruppe, oder eine carbozyklische $C_{6-10}$-Aryl- oder carbozyklische $C_{6-10}$-Aryl-$C_{1-4}$-alkylgruppe ist, gegebenenfalls an dem aromatischen Ring durch ein oder mehrere Halogenatome, $C_{1-4}$-Alkyl- oder $C_{1-4}$-Alkoxygruppen substituiert;

worin

$R^1$ und $R^2$ zusammen eine Methylengruppe sind, oder, wenn X eine >NR-Gruppe ist, kann eines von $R^1$ und $R^2$ zusätzlich eine Hydroxylgruppe und das andere eine Methylgruppe bedeuten;

worin

Z ein Wasserstoff- oder Halogenatom oder eine Trihalomethyl- oder $C_{2-4}$-Alkanoylgruppe ist;

und worin

n 2 oder 3 ist;

und optischen Isomeren und Mischungen davon und allen Säureadditions- und quartären Ammoniumsalzen davon, umfassend:

a) für die Herstellung einer Verbindung der Formel (I), worin X eine Gruppe NR ist, Reaktion eines 2-Oxo-3,8-diazaspiro[4,5]decanderivates der Formel (II)

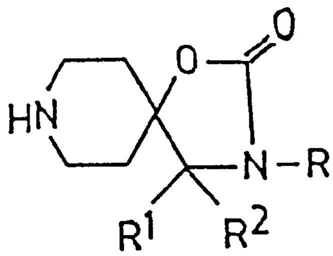

(II)

worin R, $R^1$ und $R^2$ wie oben definiert sind, mit einem Phenothiazinderivat der Formel (III)

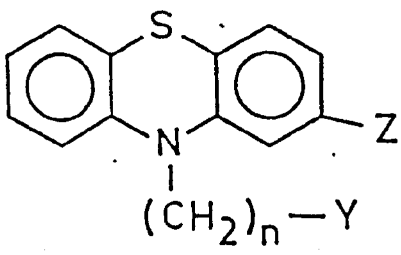

(III)

worin n und Z wie oben definiert sind und worin Y ein Halogenatom oder eine $C_{1-4}$-Alkylsulfonyloxy- oder Arylsulfonyloxygruppe ist;
oder
b) Reaktion eines 2-Oxo-3,8-diazaspiro[4,5]-decanderiviates der Formel (IV)

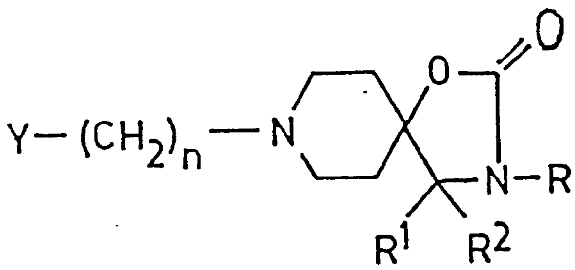

(IV)

worin R, n, $R^1$, $R^2$ und Y wie oben definiert sind, mit einem Phenothiazinderiviat der Formel (V)

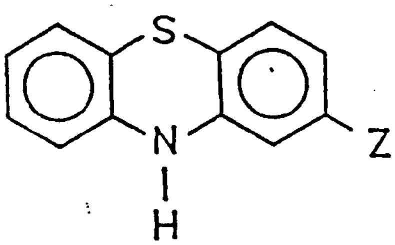

(V)

worin Z wie oben definiert ist;
oder
c) für die Herstellung von Verbindungen der Formel (I), worin $R_1$ und $R_2$ zusammen eine Methylengruppe sind, Reaktion einer Verbindung der Formel (VI)

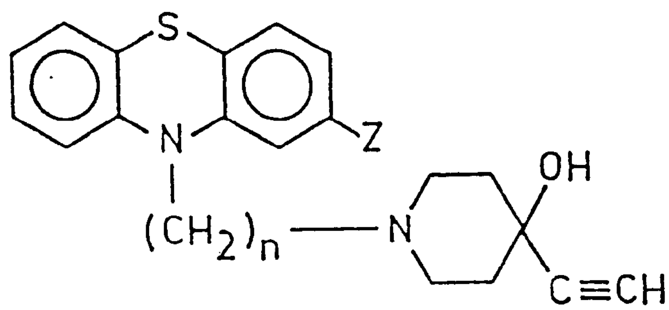

(VI)

wobei n und Z wie oben definiert sind, mit einem Isocyanat der Formel R-NCO, worin R wie oben definiert ist, unter Erhalt eines 4-Carbamoyloxy-4-ethynylpiperidinderivates der Formel (VII)

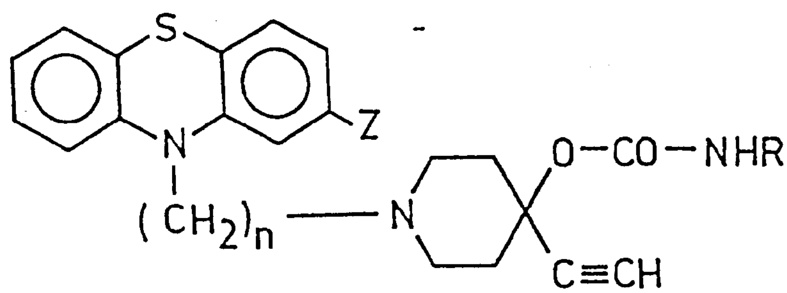

(VII)

worin R, n und Z wie oben definiert sind, gefolgt von einem der folgenden Schritte:

α) für Verbindungen, worin X ein Sauerstoffatom ist, Zyklisierung der Verbindung der Formel (VII), wie oben hergestellt und definiert, in einem sauren Medium und Reaktion des resultierenden Salzes der Formel (VIII)

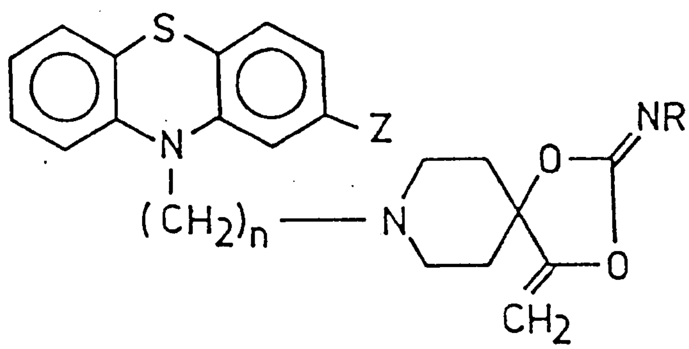

(VIII)

worin R, n und Z wie oben definiert sind, mit Wasser;

oder

β) für Verbindungen, worin X eine NR-Gruppe ist, Zyklisierung der Verbindung der Formel (VII), wie oben hergestellt und definiert, in einem basischen Medium;

oder

d) für die Herstellung von Verbindungen der Formel (I), wie im Verfahren (c) (α) definiert, Zyklisierung eines 4-Carbamoyloxy-4-ethynylpiperidinderivates der Formel (VII) wie oben definiert in einem sauren Medium und Reaktion des erhaltenen Salzes der Formel (VIII) wie oben definiert mit Wasser;

oder

e) für die Herstellung von Verbindungen der Formel (I), wie im obigen Verfahren (c) (β) definiert, Zyklisieren eines 4-Carbamoyloxy-4-ethynylpiperidinderivates der Formel (VII) wie oben definiert in einem basischen Medium;

oder

f) für die Herstellung von Verbindungen der Formel (I), worin X eine >NR-Gruppe ist, und worin eines von $R^1$ und $R^2$ eine Hydroxylgruppe und das andere eine Metyhlgruppe ist, Reaktion eines 4-Acetyl-4-hydroxypiperidinderivates der Formel (X)

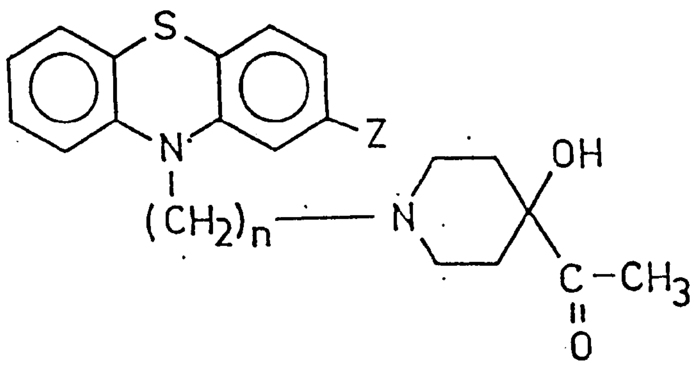

(X)

worin n und Z wie oben definiert sind, mit einem Isocyanat der Formel R-NCO, worin R wie oben definiert ist, unter Erhalt eines 4-Acetyl-4-carbamoyloxypiperidinderviates der Formel (IX)

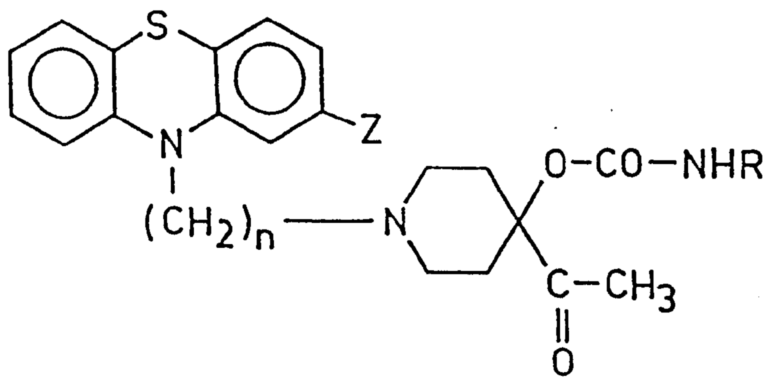

(IX)

worin R, n und Z wie oben definiert sind, das dann zyklisiert wird;
oder
g) für die Herstellung von Verbindungen der Formel (I), worin X eine >NR-Gruppe ist und worin eines von $R^1$ und $R^2$ eine Hydroxylgruppe und das andere eine Methylgruppe ist, Zyklisieren eines 4-Acetyl-4-carbamoyloxypiperidinderivates der Formel (IX) wie oben definiert;
falls erwünscht oder notwendig,
mit anschließender Reaktion einer Verbindung der Formel (I), wie oben hergestellt, worin X ein Sauerstoffatom ist und worin $R^1$ und $R^2$ zusammen eine Methylengruppe sind und worin n und Z wie oben definiert sind, mit einem Amin der Formel $R-NH_2$, worin R wie oben definiert ist, zur Herstellung der Formel (I), worin X eine NR-Gruppe ist und worin eines von $R^1$ und $R^2$ eine Hydroxylgruppe und das andere eine Methylgruppe ist;
und/oder falls gewünscht oder erforderlich, Umwandlung einer funktionellen Gruppe einer Verbindung der Formel (I), wie oben hergestellt, in eine andere Verbindung der Formel (I) wie hierin definiert;
und/oder falls erwünscht oder erfoderlich,Reaktion einer Verbindung der Formel (I), wie oben hergestellt, mit einer Säure, unter Erhalt des Säureadditionssalzes davon und/oder Reaktion einer Base mit einem Salz einer Verbindung der Formel (I) wie oben hergestellt, zur Freisetzung der freien basischen Form davon und/oder Umwandlung einer somit hergestellten Verbindung der Formel (I) in ihr quartäres Ammoniumsalz.

2. Verfahren nach Anspruch 1, worin Y entweder ein Brom- oder Chloratom oder eine p-Toluolsulfonyloxygruppe ist.

3. Verfahren nach Anspruch 1, Verfahren (c) (β), dadurch **gekennzeichnet**, daß die Reaktion als ein Einzelschritt ohne Isolierung der Verbindung der Formel (VII) durchgeführt wird.

4. Verfahren nach Anspruch 1, Verfahren (d), dadurch **gekennzeichnet**, daß die Zyklisierung des 4-Carbamoyloxy-4-ethynylpiperidinderivates der Formel (VII) in Dioxan und in der Gegenwart von Chlorwasserstoff durchgeführt wird.

5. Verfahren nach Anspruch 1, Verfahren (e), dadurch **gekennzeichnet**, daß die Zyklisierung des 4-Carbamoyloxy-4-ethynylpiperidinderivates der Formel (VII) in Methanol und in der Gegenwart von Natriummethoxid durchgeführt wird.

6. Verfahren nach Anspruch 1, zur Herstellung einer Verbindung der Formel (I) wie in Anspruch 1 definiert, ausgewählt aus der Gruppe, umfassend:
8-[3-(2-Chloro-10H-phenothiazin-10-yl)propyl]-3-methyl-4-methylen-2-oxo-1-oxa-3,8-diazaspiro[4,5]decan,
3-Methyl-4-methylen-2-oxo-8-[3-(2-trifluoromethyl-10H-phenothiazin-10-yl)propyl]-1-oxa-3,8-diazaspiro[4,5]decan,
3-Ethyl-4-methylen-2-oxo-8-[3-(2-trifluoromethyl-10H-phenothiazin-10-yl)propyl]-1-oxa-3,8-diazaspiro[4,5]decan,
8-[3-(2-Chloro-10H-phenothiazin-10-yl)propyl]-3-ethyl-4-methylen-2-oxo-1-oxa-3,8-diazaspiro[4,5]decan,
8-[3-(2-Chloro-10H-phenothiazin-10-yl)propyl]-3,4-dimethyl-4-hydroxy-2-oxo-1-oxa-3,8-diazaspiro[4,5]decan,
3,4-Dimethyl-4-hydroxy-2-oxo-8-[3-(2-trifluoromethyl-10H-phenothiazin-10-yl)propyl]-1-oxa-3,8-diazaspiro[4,5]decan,
4-Methylen-2-oxo-8-[3-(2-trifluoromethyl-10H-phenothiazin-10-yl)propyl]-1,3-dioxa-8-azaspiro[4,5]decan,
und optische Isomere und Mischungen davon und alle Säureadditions- und quartären Ammoniumsalze davon.

7. Verwendung einer Verbindung der Formel (I) nach Anspruch 1 bei der Herstellung eines Medikamentes für die Behandlung von psychiatrischen und allergischen Erkrankungen in Säugern.

8. Verfahren zur Herstellung einer pharmazeutischen Zusammensetzung, umfassend als aktiven Bestandteil eine Verbindung der Formel (I) wie in Anspruch 1 definiert, oder ein pharmazeutisch akzeptables Säureadditions- oder quartäres Ammoniumsalz davon, worin der aktive Bestandteil mit einem pharmazeutisch akzeptablen Träger und/oder Additiv vermischt wird.

## Revendications

### Revendications pour les Etats contractants suivants : AT, BE, CH, DE, DK, FR, GB, IT, LI, LU, NL, SE

1. Composé de formule (I),

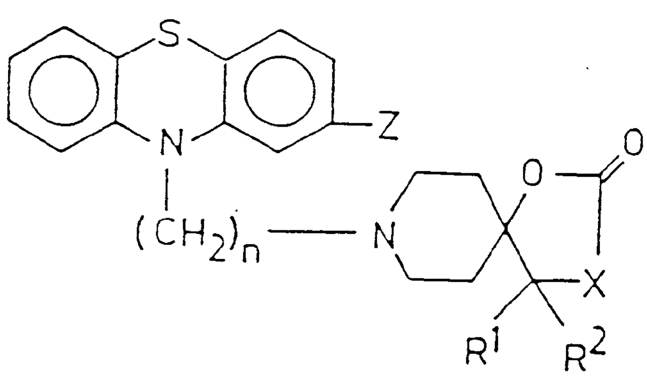

(I)

dans laquelle X représente un atome d'oxygène ou un groupe NR, dans lequel R représente un atome d'hydrogène ou un groupe alkyle en $C_{1-12}$ ou cycloalkyle en $C_{3-6}$ , ou un groupe aryle en $C_{6-10}$ carbocyclique ou aryle en $C_{6-10}$ carbocyclique -alkyle en $C_{1-4}$ éventuellement substitué sur le noyau aromatique par un ou plusieurs atomes d'halogène, ou des groupes alkyle en $C_{1-4}$ ou alcoxy en $C_{1-4}$ ;
$R^1$ et $R^2$ représentent conjointement un groupe méthylène ou lorsque X est un groupe > NR, l'un d'entre

$R^1$ et $R^2$ peut représenter en outre un groupe hydroxyle et l'autre représentant un groupe méthyle ;
Z représente un atome d'hydrogène ou d'halogéne ou un groupe trihalogénométhyle ou alcanoyle en $C_{2-4}$, et
n vaut 2 ou 3 ;
et leurs isomères optiques et leurs mélanges et tous leurs sels d'addition d'acides et d'ammonium quaternaire.

**2.** Composé de formule (I) tel que défini dans la revendication 1, choisi dans le groupe comprenant :
8-(3-(2-chloro-10H phénothiazin-10-yl)propyl)-3-méthyl-4-méthylène-2-oxo-1-oxa-3,8-diazaspiro(4,5)décane,
3-méthyl-4-méthylène-2-oxo-8-(3-(2-trifluorométhyl-10H-phénothiazin--10-yl)propyl)-1-oxa-3,8-diazaspiro(4,5)décane,
3-éthyl-4-méthylène-2-oxo-8-(3-(2-trifluorométhyl-10H-phénothiazin- -10-yl)propyl)-1-oxa-3,8-diazaspiro(4,5)décane,
8-(3-(2-chloro-10H-phénothiazin-10-yl)propyl)-3-éthyl-4-méthylène- -2-oxo-1-oxa-3,8-diazaspiro(4,5)décane,
8-(3-(2-chloro-10H-phénothiazin-10-yl)propyl)-3,4-diméthyl-4-hydroxy- -2-oxo-1-oxa-3,8-diazaspiro(4,5)décane,
3,4-diméthyl-4-hydroxy-2-oxo-8-(3-(2-trifluorométhyl-10H-phénothiazin-10-yl)propyl) -1-oxa-3,8-diazaspiro(4,5)décane,
4-méthylène-2-oxo-8-(3-(2-trifluorométhyl-10H-phénothiazin-propyl)-1,3-dioxa-8-diazaspiro(4,5)décane,
et leurs mélanges et isomères optiques et tous leurs sels d'addition d'acides et d'ammonium quaternaire.

**3.** Composition pharmaceutique, qui comprend un composé de formule (I) tel que défini dans la revendication 1 ou un de ses sels d'ammonium quaternaire ou d'addition d'acides pharmaceutiquement actifs en mélange avec un véhicule et/ou un additif pharmaceutiquement acceptable.

**4.** Procédé pour la préparation d'un composé de formule (I) tel que défini dans la revendication 1, qui comprend

a) pour la préparation d'un composé de formule (I) dans laquelle X est un groupe NR, la réaction d'un dérivé de 2-oxo-3,8-diazaspiro(4-5)décane de formule (II),

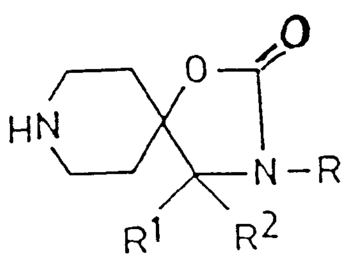

(II)

dans laquelle R, $R^1$ et $R^2$ sont tels que définis dans la revendication 1 ci-dessus, avec un dérivé de phénothiazine de formule (III),

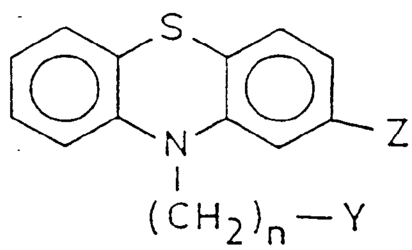

(III)

dans laquelle n et Z sont tels que définis dans la revendication 1 et Y représente un atome d'halogène ou un groupe (alkyle en $C_{1-4}$)sulfonyloxy ou arylsulfonyloxy ;
ou

b) la réaction d'un dérivé de 2-oxo-3,8-diazaspiro(4,5)décane de formule (IV),

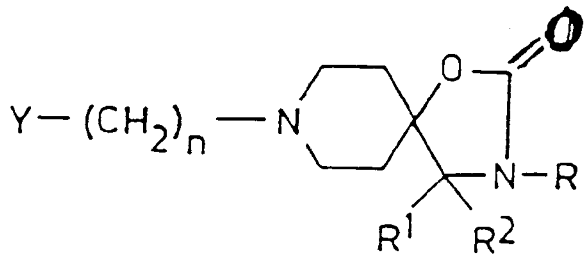

(IV)

dans laquelle R, n, $R^1$, $R^2$ et Y sont tels que définis ci-dessus, avec un dérivé de phénothiazine de formule (V),

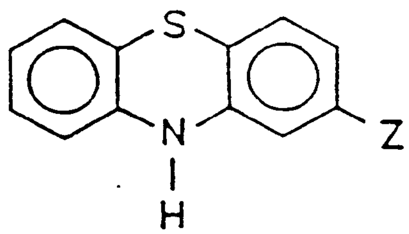

(V)

dans laquelle Z est tel que défini ci-dessus ;
ou
c) pour la préparation des composés de formule (I) dans laquelle $R_1$ et $R_2$ représentent conjointement un groupe méthylène, la réaction d'un composé de formule (VI),

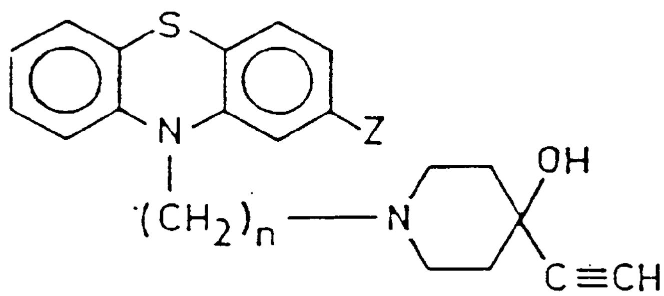

(VI)

dans laquelle n et Z sont tels que définis ci-dessus, avec un isocyanate de formule R-NCO, dans laquelle R est tel que défini ci-dessus, pour obtenir un dérivé de 4-carbamoyloxy-4-éthynyl-pipéridine de formule (VII),

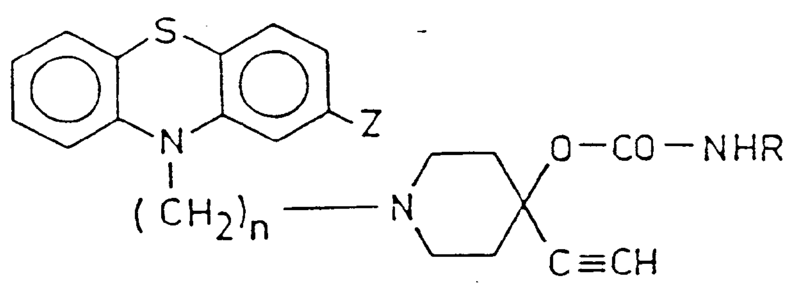

(VII)

dans laquelle R, n et Z sont tels que définis ci-dessus, suivie par soit :

α) pour les composés dans lesquels X est un atome d' oxygène, la cyclisation dans un milieu acide du composé de formule (VII) tel que préparé et défini ci-dessus et la réaction du sel résultant de formule (VIII),

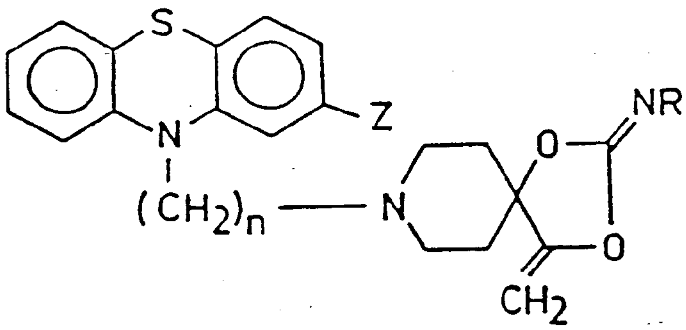

(VIII)

dans laquelle R, n et Z sont tels que définis ci-dessus, avec de l'eau ; soit

(β) pour les composés dans lesquels X est un groupe NR, la cyclisation dans un milieu basique du composé de formule (VII) tel que préparé et défini ci-dessus ;

ou

d) pour la préparation des composés de formule (I) tels que définis dans le procédé (c) (α) ci-dessus, la cyclisation dans un milieu acide d'un dérivé de 4-carbamoyloxy-4-éthynylpipéridine de formule (VII) telle que définie ci-dessus et la réaction du sel obtenu de formule (VIII) telle que définie ci-dessus avec de l'eau ;

ou

e) pour la préparation des composés de formule (I) tels que définis dans le procédé (c) (β) ci-dessus, la cyclisation dans un milieu basique d'un dérivé de 4-carbamoyloxy-4-éthynylpipéridine de formule (VII) tel que défini ci-dessus ;

ou

f) pour la préparation des composés de formule (I) dans laquelle X est un groupe >NR et l'un d'entre $R^1$ et $R^2$ est un groupe hydroxyle, l'autre étant un groupe méthyle, la réaction d'un dérivé de 4-acétyl-4-hydroxypipéridine de formule (X),

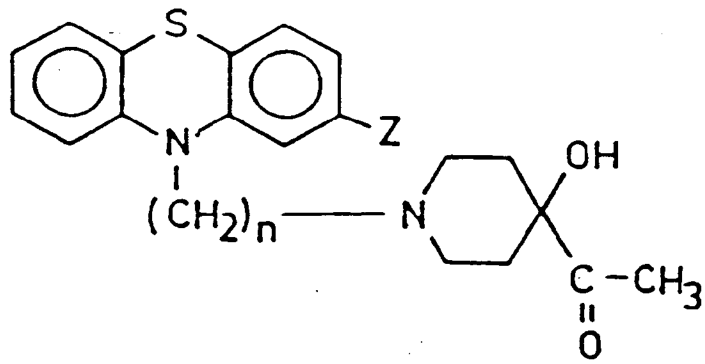

(X)

dans laquelle n et Z sont tels que définis ci-dessus, avec un isocyanate de formule R-NCO, dans laquelle R est tel que défini ci-dessus, pour obtenir un dérivé de 4-acétyl-4-carbamoyloxypipéridine de formule (IX)

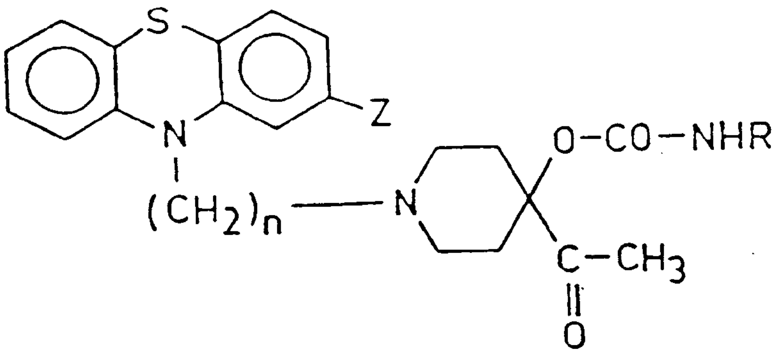

(IX)

dans laquelle R, n et Z sont tels que définis ci-dessus, que l'on cyclise ensuite ;

ou

g) pour la préparation des composés de formule (I) dans laquelle X est un groupe > NR et l'un d'entre $R^1$ et $R^2$ est un groupe hydroxyle, l'autre étant un groupe méthyle, la cyclisation d'un dérivé de 4-acétyl-4-carbamoyloxypipéridine de formule (IX) tel que défini ci-dessus ;

suivie si nécessaire ou si on le souhaite,

par la réaction d'un composé de formule (I) tel que préparé ci-dessus, dans lequel X est un atome d'oxygène et $R^1$ et $R^2$ représentent conjointement un groupe méthylène et n et Z sont tels que définis ci-dessus, avec une amine de formule $R\text{-}NH_2$, dans laquelle R est tel que défini ci-dessus, afin de préparer un composé de formule (I), dans laquelle X est un groupe NR et l'un d'entre $R^1$ et $R^2$ est un groupe hydroxyle, l'autre étant un groupe méthyle ;

et/ou si on le souhaite ou si nécessaire,

la transformation d'un groupe fonctionnel d'un composé de formule (I) tel que préparé ci-dessus en un autre composé de formule (I) tel que défini dans la revendication ;

et/ou si on le souhaite ou si nécessaire,

la réaction d'un composé de formule (I) tel que préparé ci-dessus, avec un acide pour obtenir son sel d'addition d'acide et/ou la réaction d'une base avec un sel d'un composé de formule (I) tel que préparé ci-dessus pour libérer sa forme de base libre et/ou la conversion d'un composé de formule (I) ainsi préparé en son sel d'ammonium quaternaire.

5. Procédé selon la revendication 4, dans lequel Y est soit un atome de brome ou de chlore soit un groupe p-toluènesulfonyloxy.

6. Procédé selon la revendication 4, procédé (c) (β), dans lequel on effectue la réaction sous la forme d'une seule étape sans isoler le composé de formule (VII).

7. Procédé selon la revendication 4, procédé (d), dans lequel on effectue la cyclisation du dérivé de 4-carbamoyloxy-éthynylpypéridine de formule (VII) dans du dioxanne et en présence de chlorure d'hydrogène.

8. Procédé selon la revendication 4, procédé (e), dans lequel on effectue la cyclisation du dérivé de 4-carbamoyloxy-4-éthynylpipéridine de formule (VII) dans du méthanol et en présence de méthylate de sodium.

9. Utilisation d'un composé de formule (I) tel que défini dans la revendication 1, dans la préparation d'un médicament pour le traitement de maladies psychiatriques et allergiques chez les mammifères.

10. Procédé pour la préparation d'une composition pharmaceutique telle que définie dans la revendication 3, dans lequel on mélange un composé de formule (I) tel que défini dans la revendication 1 ou un de ses sels d'ammonium quaternaire ou d'addition d'acides pharmaceutiquement acceptables avec un véhicule et/ou un additif pharmaceutiquement acceptable.

**Revendications pour les Etats contractants suivants : ES, GR**

1. Procédé de préparation d'un composé de formule (I),

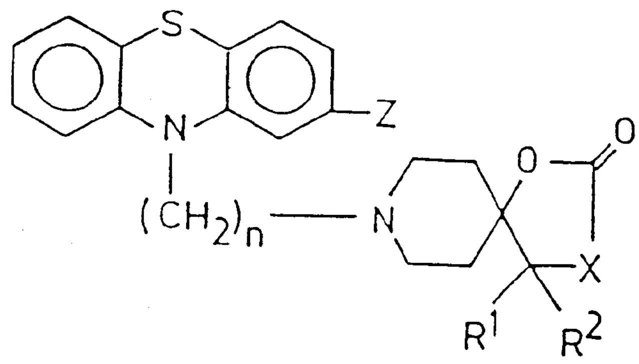

( I )

dans laquelle X représente un atome d'oxygène ou un groupe NR, dans lequel R représente un atome d'hydrogène ou un groupe alkyle en $C_{1-12}$ ou cycloalkyle en $C_{3-6}$ , on un groupe aryle en $C_{6-10}$ carbocyclique ou aryle en $C_{6-10}$ carbocyclique -alkyle en $C_{1-4}$ éventuellement substitué sur le noyau aromatique par un ou plusieurs atomes d'halogène, ou des groupes alkyle en $C_{1-4}$ ou alcoxy en $C_{1-4}$ ;
$R^1$ et $R^2$ représentent conjointement un groupe méthylène ou lorsque X est un groupe > NR, l'un d'entre $R^1$ et $R^2$ peut représenter en outre un groupe hydroxyle et l'autre représentant un groupe méthyle ;
Z représente un atome d'hydrogène ou d'halogéne ou un groupe trihalogénométhyle ou alcanoyle en $C_{2-4}$, et
n vaut 2 ou 3 ;
et de ses isomères optiques et leurs mélanges et de tous ses sels d'addition d'acides et d'ammonium quaternaire, qui comprend

a) pour la préparation d'un composé de formule (I) dans laquelle X est un groupe NR, la réaction d'un dérivé de 2-oxo-3,8-diaza - spiro(4-5)décane de formule (II),

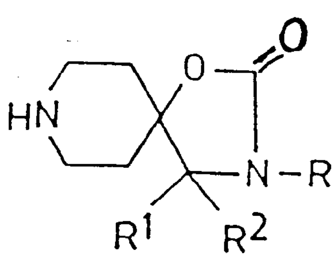

( II )

dans laquelle R, $R^1$ et $R^2$ sont tels que définis ci-dessus, avec un dérivé de phénothiazine de formule (III),

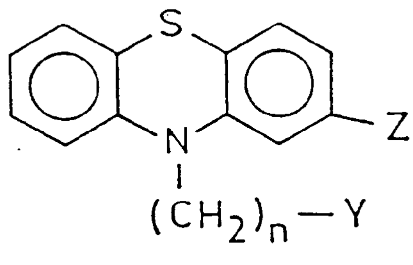

( III)

dans laquelle n et Z sont tels que définis ci-dessus et Y représente un atome d'halogène ou un groupe (alkyle en $C_{1-4}$)-sulfonyloxy ou arylsulfonyloxy ;
ou
b) la réaction d'un dérivé de 2-oxo-3,8-diazaspiro(4,5)décane de formule (IV),

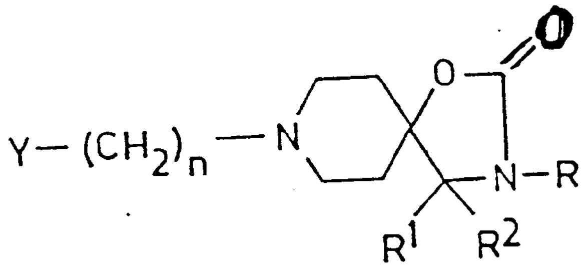

(IV)

dans laquelle R, n, $R^1$, $R^2$ et Y sont tels que définis ci-dessus, avec un dérivé de phénothiazine de formule (V),

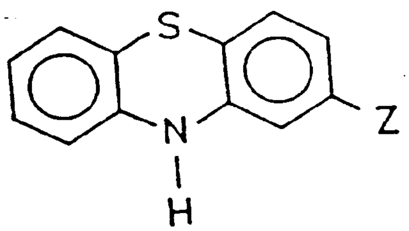

(V)

dans laquelle Z est tel que défini ci-dessus ;
ou
c) pour la préparation des composés de formule (I) dans laquelle $R_1$ et $R_2$ représentent conjointement un groupe méthylène, la réaction d'un composé de formule (VI),

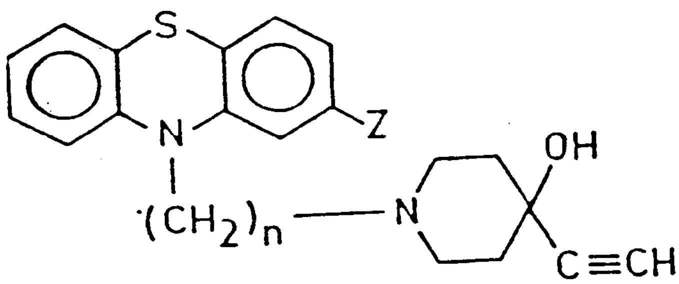

(VI)

dans laquelle n et Z sont tels que définis ci-dessus, avec un isocyanate de formule R-NCO, dans laquelle R est tel que défini ci-dessus, pour obtenir un dérivé de 4-carbamoyloxy-4-éthynylpipéridine de formule (VII),

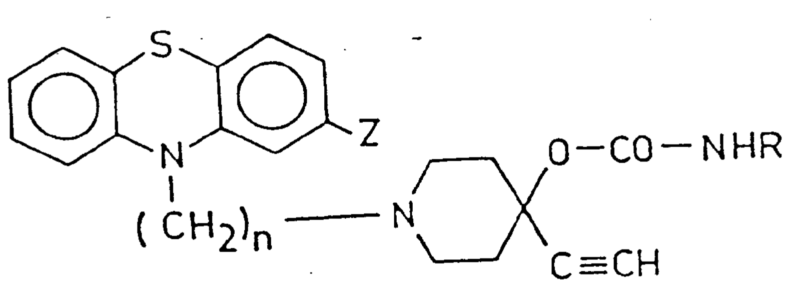

(VII)

dans laquelle R, n et Z sont tels que définis ci-dessus, suivie par soit :
α) pour les composés dans lesquels X est un atome d' oxygène, la cyclisation dans un milieu acide du composé de formule (VII) tel que préparé et défini ci-dessus et la réaction du sel résultant de formule (VIII),

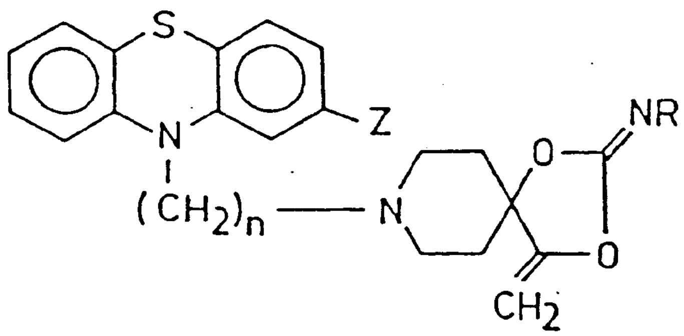

(VIII)

dans laquelle R, n et Z sont tels que définis ci-dessus, avec de l'eau ; soit

β) pour les composés dans lesquels X est un groupe NR, la cyclisation dans un milieu basique du composé de formule (VII) tel que préparé et défini ci-dessus ;

ou

d) pour la préparation des composés de formule (I) tels que définis dans le procédé (c) (α) ci-dessus, la cyclisation dans un milieu acide d'un dérivé de 4-carbamoyloxy-4-éthynylpipéridine de formule (VII) telle que définie ci-dessus et la réaction du sel obtenu de formule (VIII) telle que définie ci-dessus avec de l'eau ;

ou

e) pour la préparation des composés de formule (I) tels que définis dans le procédé (c) ( (β) ci-dessus, la cyclisation dans un milieu basique d'un dérivé de 4-carbamoyloxy-4-éthynylpipéridine de formule (VII) tel que défini ci-dessus ;

ou

f) pour la préparation des composés de formule (I) dans laquelle X est un groupe > NR et l'un d'entre $R^1$ et $R^2$ est un groupe hydroxyle, l'autre étant un groupe méthyle, la réaction d'un dérivé de 4-acétyl-4-hydroxypipéridine de formule (X),

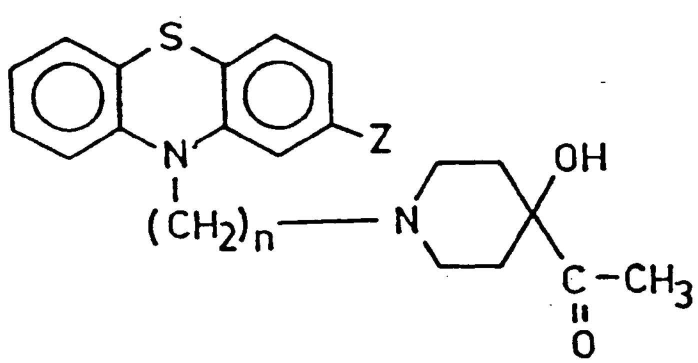

(X)

dans laquelle n et Z sont tels que définis ci-dessus, avec un isocyanate de formule R-NCO, dans laquelle R est tel que défini ci-dessus, pour obtenir un dérivé de 4-acétyl-4-carbamoyloxypi- péridine de formule (IX)

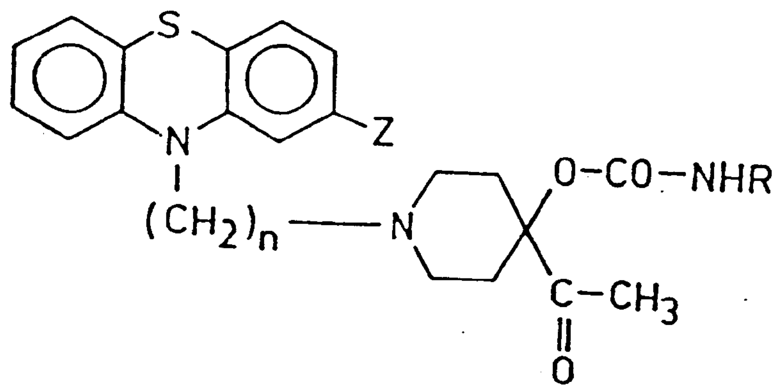

(IX)

dans laquelle R, n et Z sont tels que définis ci-dessus, que l'on cyclise ensuite ;

ou

g) pour la préparation des composés de formule (I) dans laquelle X est un groupe > NR et l'un d'entre $R^1$ et $R^2$ est un groupe hydroxyle, l'autre étant un groupe méthyle, la cyclisation d'un dérivé de 4-acétyl-4-carbamoyloxypipéridine de formule (IX) tel que défini ci-dessus ;

suivie si nécessaire ou si on le souhaite,

par la réaction d'un composé de formule (I) tel que préparé ci-dessus, dans lequel X est un atome d'oxygène et $R^1$ et $R^2$ représentent conjointement un groupe méthylène et n et Z sont tels que définis ci-dessus, avec une amine de formule $R\text{-}NH_2$, dans laquelle R est tel que défini ci-dessus, afin de préparer un composé de formule (I), dans laquelle X est un groupe NR et l'un d'entre $R^1$ et $R^2$ est un groupe hydroxyle, l'autre étant un groupe méthyle ;

et/ou si on le souhaite ou si nécessaire,

la transformation d'un groupe fonctionnel d'un composé de formule (I) tel que préparé ci-dessus en un autre composé de formule (I) tel que défini ci-dessus;

et/ou si on le souhaite ou si nécessaire,

la réaction d'un composé de formule (I) tel que préparé ci-dessus, avec un acide pour obtenir son sel d'addition d'acide et/ou la réaction d'une base avec un sel d'un composé de formule (I) tel que préparé ci-dessus pour libérer sa forme de base libre et/ou la conversion d'un composé de formule (I) ainsi préparé en son sel d'ammonium quaternaire.

**2.** Procédé selon la revendication 1, dans lequel Y est soit un atome de brome ou de chlore soit un groupe p-toluènesulfonyloxy.

**3.** Procédé selon la revendication 1, procédé (c) (β), dans lequel on effectue la réaction sous la forme d'une seule étape sans isoler le composé de formule (VII).

**4.** Procédé selon la revendication 1, procédé (d), dans lequel on effectue la cyclisation du dérivé de 4-carbamoyloxy-éthynylpypéridine de formule (VII) dans du dioxanne et en présence de chlorure d'hydrogène.

**5.** Procédé selon la revendication 1, procédé (e), dans lequel on effectue la cyclisation du dérivé de 4-carbamoyloxy-4-éthynylpipéridine de formule (VII) dans du méthanol et en présence de méthylate de sodium.

**6.** Procédé selon la revendication 1, pour la préparation d'un composé de formule (I) tel que défini dans la revendication 1, choisi dans le groupe comprenant :

8-(3-(2-chloro-10H-phénothiazin-10-yl)propyl)-3-méthyl-4-méthylène-2-oxo-1-oxa-3,8-diazaspiro(4,5) décane

3-méthyl-4-méthylène-2-oxo-8-(3-(2-trifluorométhyl-10H-phénothiazin- -10-yl)propyl)-1-oxa-3,8-diazaspiro(4,5)décane

3-éthyl-4-méthylène-2-oxo-8-(3-(2-trifluorométhyl-10H-phénothiazin--10-yl)propyl)-1-oxa-3,8-diazaspiro(4,5)décane

8-(3-(2-chloro-10H-phénothiazin-10-yl)propyl)-3-éthyl-4-méthylène-2-oxo-1-oxa-3,8-diazaspiro(4,5)décane

8-(3-(2-chloro-10H-phénothiazin-10-yl)propyl)-3,4-diméthyl-4-hydroxy-2-oxo-1-oxa-3,8-diazaspiro(4,5)décane

3,4-diméthyl-4-hydroxy-2-oxo-8-(3-(2-trifluorométhyl-10H-phénothiazin-10-yl)propyl) -1-oxa-3,8-diazaspiro(4,5)décane
4-méthylène-2-oxo-8-(3-(2-trifluorométhyl-10H-phénothiazin-10-yl)-propyl)-1,3-dioxa-8-diazaspiro(4,5)décane, et leurs mélanges et isomères optiques et tous leurs sels d'ammonium quaternaire et d'addition d'acides.

7. Utilisation d'un composé de formule (I) tel que défini dans la revendication 1, dans la préparation d'un médicament pour le traitement des maladies psychiatriques et allergiques chez les mammifères.

8. Procédé de préparation d'une composition pharmaceutique qui comprend, à titre de principe actif, un composé de formule (I) tel que défini dans la revendication 1 ou un de ses sels d'ammonium quaternaire ou d'addition d'acides pharmaceutiquement acceptables, dans lequel on mélange le principe actif avec un véhicule et/ou un additif pharmaceutiquement acceptable.